# EUROPEAN PATENT APPLICATION

(11) **EP 3 617 222 A1**
(43) Date of publication of application: **04.03.2020**
(21) Application number: 18791294.4
(22) Date of filing: 24.04.2018
(51) Int. Cl.: C07K 14/00, A61K 38/16, A61P 43/00

(54) **PEPTIDE FOR INDUCING REGENERATION OF TISSUE, AND USE THEREOF**

(30) Priority: 25.04.2017 JP 2017085793
(71) Applicant: Shionogi & Co., Ltd, Osaka-shi, Osaka 541-0045 (JP); Stemrim Inc., Ibaraki-shi, Osaka 567-0085 (JP)
(72) Inventor: NIHASHI, Yoichiro, Toyonaka, Osaka 561-0825 (JP); KAWACHI, Tomoyuki, Toyonaka, Osaka 561-0825 (JP); YAMATSU, Yukiko, Toyonaka, Osaka 561-0825 (JP); FUMOTO, Masataka, Toyonaka, Osaka 561-0825 (JP); YAMAZAKI, Takehiko, Ibaraki-shi Osaka 567-0085 (JP)
(74) Representative: Vossius & Partner Patentanwälte Rechtsanwälte mbB
(86) International application number: PCT/JP2018/016654
(87) International publication number: WO 2018/199107

(57) **Abstract**

A peptide which can be utilized as an active ingredient of a composition for use in stimulation of migration of PDGFRα-positive cells, a composition for use in recruitment of bone marrow mesenchymal stem cells from bone marrow to peripheral blood, a composition for use in regeneration of tissues, or a composition for use in stimulation of migration of bone marrow-derived stromal cells is provided.

## Description

### TECHNICAL FIELD

The present invention relates to a peptide for inducing regeneration of tissues, and uses thereof.

### BACKGROUND ART

It has become even clearer that each of organs and tissues in the living bodies has tissue stem cells maintaining its structural and functional homeostasis. For example, the myocardial stem cells exist in the heart, the neural stem cells exist in the brain, and the epidermal stem cells and follicular stem cells exist in the skin, which supply myocardial cells, neural cells, epidermal cells, and follicular epithelial cells over the lifetime to maintain the structures and functions of the heart, the brain, and the skin. Meanwhile, the hematopoietic stem cells that differentiate into blood cells such as red blood cells, white blood cells, and platelets exist in the bone marrow.

These blood cells derived from the hematopoietic stem cells circulate all sorts of organs and tissues in the bodies through blood streams, and play some indispensable functions for life maintenance such as oxygen supply, immune responses, and hematostatis or recovery of damaged tissues. In other words, it is fair to say that the bone marrow hematopoietic stem cells contribute to the maintenance of homeostasis of all the tissues in the live bodies via peripheral circulation, rather than the maintenance of homeostasis of the bone marrow or the bone tissues in which the bone marrow hematopoietic stem cells are localized.

In the recent years, it has been clarified that in addition to the hematopoietic stem cells, the mesenchymal stem cells that are differentiable into not only mesodermal tissues of bones, cartilages, adipose or the like, but also ectodermal tissues such as nerves and epidermides exist in the bone marrow; however, the existing significance in the live bodies has not yet been clarified in many ways. However, if the hematopoietic stem cells that maintain homeostasis of all the tissues and organs by supplying blood cells via the peripheral circulation exist in the bone marrow, there is similarly an expected possibility that the mesenchymal stem cells that exist in the bone marrow would also contribute to the maintenance of homeostasis of the tissues in the live bodies by providing cells that can differentiate into bones, cartilages, adipose, nerves, epithelia, or the like via the peripheral circulation to the tissues or organs in the live bodies in need thereof.

Currently, regenerative medicine has been actively developed, in which bone marrow mesenchymal stem cells are prepared by collection of the bone marrow blood, expanded by cell culture, and grafted to a damaged site of the intractable tissues or into the peripheral circulation blood to induce the regeneration of damaged tissues. The clinical applications of transplantation of the bone marrow mesenchymal stem cells have been already been underway in the regenerative medicine of cerebral infarction, myocardial infarction, intractable skin ulcer or the like. Further, it has been clarified that the transplanted bone marrow mesenchymal stem cells exhibit an action of suppressing inflammations and immune responses and an action of suppressing fibrous scar formation locally in the live bodies, so that the clinical tests of the bone marrow mesenchymal stem cells transplantation therapy have been started as a new therapeutic method for graft versus host disease (GVHD), which is a serious side reaction after the bone marrow transplantation or transfusion, or for scleroderma, which is an autoimmune disease. However, the bone marrow blood containing the bone marrow mesenchymal stem cells is only collected by an invasive method in which thick needles are repeatedly pierced to the iliac bone. In addition, the bone marrow mesenchymal stem cells would gradually lose proliferation ability and pluripotency if the subculture is continued *ex vivo.* Further, the culture of the bone marrow mesenchymal stem cells based on the high-quality management that ensures safety of the *in vivo* transplantation would necessitate a specialized culturing equipment such as CPC (cell processing center), so that the cell culture is enabled at very limited universities or business enterprises at present. In other words, in order to make the regenerative medicine using the bone marrow mesenchymal stem cells available to many patients in the world suffering from intractable tissue damages, it is an urgent task to develop techniques for the regenerative medicine with the mesenchymal stem cells to be enabled in all sorts of medical facilities.

HMGB1 (High mobility group box 1: high mobility group box 1 protein) was identified about 30 years ago as a non-histone chromatin protein that controls the gene expression or repair of DNA by controlling an intranuclear chromatin structure. The structure of the HMGB1 protein is constituted mainly by two DNA binding domains, in which a DNA binding domain at an N-terminal side is called an A-box, and a DNA binding domain at a C-terminal side is called a B-box. According to the past studies, it has been clarified that a domain that binds to TLR in the HMGB1 molecule and evokes an inflammatory reaction exists within the B-box.

It has been reported that the PDGFRα-positive cells are recruited in the blood from the bone marrow by administering mice generated with skin ulcer or cerebral infarction with a recombinant HMGB 1 protein from tail vein, and further the cells are accumulated in the skin ulcer portion or the cerebral infarction portion, thereby strongly inducing the regeneration of skin ulcer or cerebral infarction. It has been already reported that the PDGFRα-positive cells in the bone marrow are mesenchymal stem cells that are differentiable into bones, cartilages, adipose, and further to nerves or epithelia. In other words, the PDGFRα-positive mesenchymal stem cells in the bone marrow are recruited to peripheral circulation by the HMGB 1 administration, thereby making it possible to accumulate many of the mesenchymal stem cells in the damaged tissues in the live bodies without extracorporeally taking the cells, and subjecting them to a specialized culturing.

The regeneration-inducing medicine can be enabled with the bone marrow mesenchymal stem cells in all sorts of medical facilities by developing HMGB 1 as a medicament for inducing regeneration of damaged tissues by the recruitment in blood of the bone marrow mesenchymal stem cells in the live bodies. As a result, many of the disadvantages faced in the present regenerative medicine using the bone marrow mesenchymal stem cells would be overcome.

As mentioned above, the HMGB1 medicament is an epoch-making therapeutic agent which promotes the recruitment of the bone marrow mesenchymal stem cells in blood or the accumulation to damaged tissues, thereby inducing regeneration of tissues in the body. Regarding HMGB1, it has been already reported that not only a full length of HMGB 1 protein but also fragments of the HMGB 1 protein has a similar action.

### PRIOR ART REFERENCES

### PATENT PUBLICATIONS

Patent Publication 1: WO 2008/053892
Patent Publication 2: WO 2007/015546
Patent Publication 3: WO 2009/133939
Patent Publication 4: WO 2009/133943
Patent Publication 5: WO 2009/133940
Patent Publication 6: Japanese Unexamined Patent Publication No. 2005-537253
Patent Publication 7: WO 2012/147470

### NON-PATENT PUBLICATIONS

Non-Patent Publication 1: Bustin et al., Mol Cell Biol, 19:5237-5246, 1999
Non-Patent Publication 2: Hori et al., J. Biol. Chem., 270, 25752-25761, 1995
Non-Patent Publication 3: Wang et al., Science, 285:248-251, 1999
Non-Patent Publication 4: Muller et al., EMBO J, 20:4337-4340, 2001
Non-Patent Publication 5: Wang et al., Science, 285:248-251, 1999
Non-Patent Publication 6: Germani et al., J Leukoc Biol. Jan; 81(1):41-5, 2007
Non-Patent Publication 7: Palumbo et al., J. Cell Biol., 164:441-449, 2004
Non-Patent Publication 8: Merenmies et al., J. Biol. Chem., 266:16722-16729, 1991
Non-Patent Publication 9: Wu Y et al., Stem cells, 25:2648-2659, 2007
Non-Patent Publication 10: Tamai et al., Proc Natl Acad Sci USA. 2011 Apr 4. [Epub ahead of print], 108:6609-6614, 2011
Non-Patent Publication 11: Yang et al., J Leukoc Biol. Jan; 81(1):59-66, 2007

### SUMMARY OF THE INVENTION

### PROBLEMS TO BE SOLVED BY THE INVENTION

An object to be solved by the present invention is to provide a peptide having activity of migrating bone marrow-derived stromal cells, in other words, a peptide for inducing regeneration of tissues.

### MEANS TO SOLVE THE PROBLEMS

As a result of intensive studies, the present inventors have found out that a peptide having substitution of an amino acid of position 43 and/or position 44 in the peptide consisting of amino acids of position 1 to position 44 of the HMGB1 protein, and a peptide having substitution of an amino acid of position 43 and/or position 44 in the peptide consisting of the amino acids of position 2 to position 44 of the HMGB 1 protein have the activity of migrating stromal cells derived from murine bone marrow, and the present invention has been perfected thereby.

Concretely, the present invention relates to:
(1) a peptide selected from the group consisting of:
   a peptide having the amino acid sequence shown in any one of SEQ ID NOs: 1 to 399 and 403,
   a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 399 and 403, and having an activity of stimulating migration of cells,
   a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 399 or an amino acid of position 1 to position 41 of the peptide having the amino acid sequence shown in SEQ ID NO: 403, and having an activity of stimulating migration of cells, a derivative thereof, a pharmaceutically acceptable salt thereof, or a solvate thereof;
(2) a peptide selected from the group consisting of:
   a peptide having the amino acid sequence shown in any one of SEQ ID NOs: 1 to 5, 126, 128, 129, 138, 167, 248, 254, 280, 282, 283, 292 and 403,
   a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 5, 126, 128, 129, 138, 167, 248, 254, 280, 282, 283, 292 and 403, and having an activity of stimulating migration of cells, and
   a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 5, 126, 128, 129, 138, 167, 248, 254, 280, 282, 283 and 292, or an amino acid of position 1 to position 41 of the peptide having the amino acid sequence shown in SEQ ID NO: 403, and having an activity of stimulating migration of cells,
   a derivative thereof, a pharmaceutically acceptable salt thereof, or a solvate thereof;
(3) a peptide selected from the group consisting of:
   a peptide having the amino acid sequence shown in any one of SEQ ID NOs: 1 to 5, 167 and 403,
   a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 5, 167 and 403, and having an activity of stimulating migration of cells, and
   a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 5 and 167 or at an amino acid of position 1 to position 41 of the peptide having the amino acid sequence shown in SEQ ID NO: 403, and having an activity of stimulating migration of cells,
   a derivative thereof, a pharmaceutically acceptable salt thereof, or a solvate thereof;
(4) a peptide selected from the group consisting of:
   a peptide having the amino acid sequence shown in any one of SEQ ID NOs: 1 to 5 and 403,
   a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 5 and 403, and having an activity of stimulating migration of cells, and
   a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 5 or at an amino acid of position 1 to position 41 of the peptide having the amino acid sequence shown in SEQ ID NO: 403, and having an activity of stimulating migration of cells, a derivative thereof, a pharmaceutically acceptable salt thereof, or a solvate thereof;
(5) a composition for use in stimulation of migration of PDGFRα-positive cells, containing a peptide, a derivative thereof, a pharmaceutically acceptable salt thereof, or a solvate thereof as defined in any one of the above (1) to (4);
(6) a composition for use in recruitment of bone marrow mesenchymal stem cells from bone marrow to peripheral blood, containing a peptide, a derivative thereof, a pharmaceutically acceptable salt thereof, or a solvate thereof as defined in any one of the above (1) to (4);
(7) a composition for use in regeneration of tissues, containing a peptide, a derivative thereof, a pharmaceutically acceptable salt thereof, or a solvate thereof as defined in any one of the above (1) to (4); and
(8) a composition for use in stimulation of migration of bone marrow-derived stromal cells, containing a peptide, a derivative thereof, a pharmaceutically acceptable salt thereof, or a solvate thereof as defined in any one of the above (1) to (4).

### ADVANTAGEOUS EFFECTS OF THE INVENTION

The peptide of the present invention can be utilized as an active ingredient of, for example, a composition for use in stimulation of migration of PDGFRα-positive cells, a composition for use in recruitment of bone marrow mesenchymal stem cells from bone marrow to peripheral blood, a composition for use in regeneration of tissues, or a composition for use in stimulation of migration of bone marrow-derived stromal cells.

### BRIEF DESCRIPTION OF THE DRAWINGS

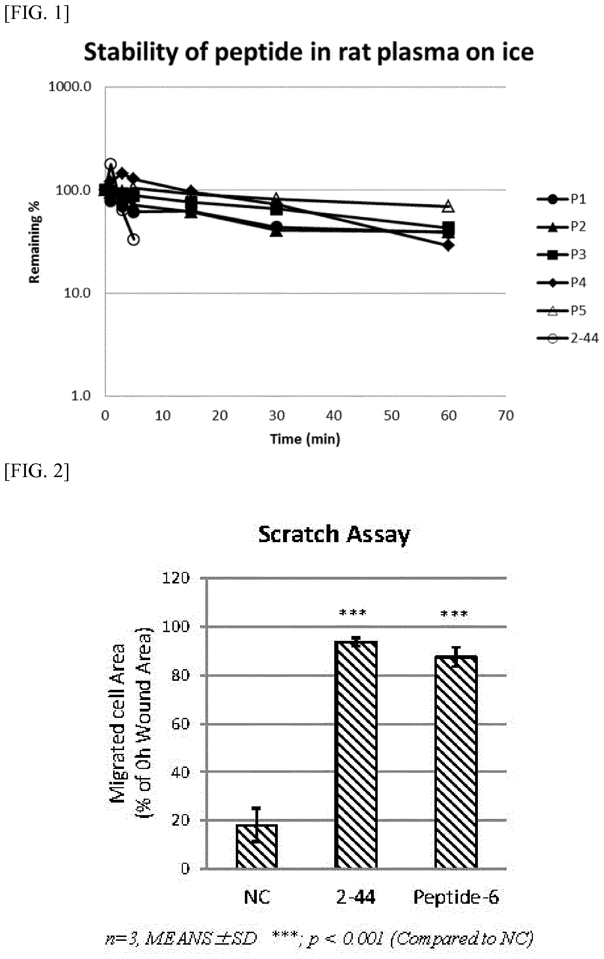

[FIG. 1] FIG. 1 is a graph showing the results of the stability test of the peptides in plasma for six kinds of peptides (a peptide of position 2 to position 44 of human HMGB1 (2-44), and five kinds of peptides in which amino acids at position 43 or/and position 44 are modified (Peptide-1 to -5)). P1 to P5 mean the peptides of Peptide-1 to -5. The axis of ordinates is the remaining rate when the peptide immediately after the addition of the plasma was calculated as 100, and the axis of the abscissas is the time after the addition of the plasma. Here, the sample of 15 minutes or later after the addition of the plasma of the peptide of 2-44 was lower than the quantifiable lower limit (0.5 µg/mL), so that the remaining rate could not be plotted in FIG. 1.
[FIG. 2] FIG. 2 is a graph of the results of the measurements of the cell-migration activity of two kinds of peptides (a peptide of position 2 to position 44 of human HMGB1 (2-44), and a peptide in which the amino acids at position 43 and position 44 are modified (Peptide-6)). NC is a negative control.

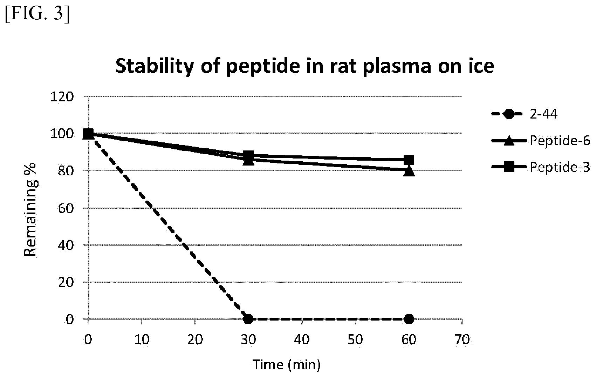

[FIG. 3] FIG. 3 is a graph showing the results of the stability test in plasma of three kinds of peptides (a peptide of position 2 to position 44 of human HMGB1 (2-44), and two kinds of peptides in which amino acids at position 43 and position 44 are modified (Peptide-3 and -6)). The axis of ordinates is the remaining rate when the peptide immediately after the addition of the plasma was calculated as 100, and the axis of the abscissas is the time after the addition of the plasma.

### MODES FOR CARRYING OUT THE INVENTION

The present invention provides a peptide as defined in any one of (a) to (c) given below:
(a) a peptide having the amino acid sequence shown in any one of SEQ ID NOs: 1 to 399 and 403;
(b) a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 399 and 403, and having an activity of stimulating migration of cells; and
(c) a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 399 or an amino acid of position 1 to position 41 of the peptide having the amino acid sequence shown in SEQ ID NO: 403, and having an activity of stimulating migration of cells.

The present invention provides a composition for use in stimulation of migration of PDGFRα-positive cells or bone marrow-derived stromal cells, containing a peptide as defined in (a) to (c) given below:
(a) a peptide having the amino acid sequence shown in any one of SEQ ID NOs: 1 to 399 and 403;
(b) a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 399 and 403, and having an activity of stimulating migration of cells; and
(c) a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 399 or an amino acid of position 1 to position 41 of the peptide having the amino acid sequence shown in SEQ ID NO: 403, and having an activity of stimulating migration of cells.

The composition for use in stimulation of migration of PDGFRα-positive cells or bone marrow-derived stromal cells of the present invention includes a reagent composition and a pharmaceutical composition. The reagent composition as used herein is also expressed as a reagent, and the pharmaceutical composition is also expressed as a medicament, a pharmaceutical agent, or a pharmaceutical composition.

The reagent composition for use in stimulation of migration of PDGFRα-positive cells or bone marrow-derived stromal cells of the present invention can be used as, for example, reagents that are necessary for basic researches and clinical researches in the developments of regenerative medicine and regeneration-inducing medicine. For example, cells are recruited in tissues in the live bodies in experimental animals by the use of the reagent composition, thereby making it possible to study the levels of tissue repair and tissue function reconstruction. Also, for example, the researches on inducing regeneration of tissues can be carried out by the recruitment of the cells *in vitro* by the use of the reagent composition.

In addition, the pharmaceutical composition for use in stimulation of migration of PDGFRα-positive cells or bone marrow-derived stromal cells of the present invention can be used as, for example, a medicament in the regenerative medicine and the regeneration-inducing medicine. For example, the tissues can be regenerated by the use of the pharmaceutical composition. Also, for example, the pharmaceutical composition can prevent the lowering of the functions of tissues and organs by the decrease in the tissue stem cells as a so-called prophylactic medicament, or can delay the progress of aging changes as an anti-aging medicament.

Here, the composition for use in stimulation of migration of PDGFRα-positive cells or bone marrow-derived stromal cells as used herein is also expressed as an agent for use in stimulation of migration of PDGFRα-positive cells or bone marrow-derived stromal cells, a stimulation agent for cell migration, a composition for use in induction of cell migration, an agent for use in induction of cell migration, an inducing agent for cell migration, or an inducing agent of cells.

In the present invention the cell migration-stimulating activity means an activity of stimulating the cell migration. The cell migration-stimulating activity as used herein is also expressed as cell migration-inducing activity or cell-inducing activity.

The present invention provides a composition for use in recruitment of bone marrow mesenchymal stem cells from bone marrow to peripheral blood, containing a substance as defined in any one of the following (a) to (c):
(a) a peptide having the amino acid sequence shown in any one of SEQ ID NOs: 1 to 399 and 403;
(b) a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 399 and 403, and having an activity of stimulating migration of cells; and
(c) a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 399 or an amino acid of position 1 to position 41 of the peptide having the amino acid sequence shown in SEQ ID NO: 403, and having an activity of stimulating migration of cells.

The composition for use in recruitment of the bone marrow mesenchymal stem cells from bone marrow to peripheral blood of the present invention includes a reagent composition and a pharmaceutical composition.

The reagent composition for use in regeneration of tissues of the present invention can be used as, for example, reagents that are necessary for basic researches and clinical researches in the developments of regenerative medicine and regeneration-inducing medicine. In addition, the pharmaceutical composition for use in regeneration of tissues of the present invention can be used as, for example, a medicament in the regenerative medicine and the regeneration-inducing medicine. For example, the bone marrow cells are recruited to the peripheral circulation by the use of the pharmaceutical composition, so that the tissues can be regenerated. Also, for example, it is possible that the cells which are recruited to the peripheral blood by utilizing the pharmaceutical composition are collected extracorporeally, concentrated, and administered to tissues to carry out the treatment. In the conventional methods, since the cells are collected from the bone marrow at the deep part in the body, the live bodies are subjected to invasiveness. However, when the pharmaceutical composition of the present invention is used, the bone marrow cells are collected from the peripheral blood at low invasion, which can be utilized in bone marrow cell transplantation or the like. Here, the composition for use in recruitment of the bone marrow mesenchymal stem cells from bone marrow to peripheral blood is also expressed as a composition for use in induction of the bone marrow mesenchymal stem cells from bone marrow to peripheral blood.

The present invention provides a composition for use in regeneration of tissues, containing a substance as defined in any one of the following (a) to (c):
(a) a peptide having the amino acid sequence shown in any one of SEQ ID NOs: 1 to 399 and 403;
(b) a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 399 and 403, and having an activity of stimulating migration of cells; and
(c) a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 399 or an amino acid of position 1 to position 41 of the peptide having the amino acid sequence shown in SEQ ID NO: 403, and having an activity of stimulating migration of cells.

The composition for use in regeneration of tissues of the present invention includes a reagent composition and a pharmaceutical composition.

The reagent composition for use in regeneration of tissues of the present invention can be used as, for example, reagents that are necessary for basic researches and clinical researches in the developments of regenerative medicine and regeneration-inducing medicine. In addition, the pharmaceutical composition for use in regeneration of tissues of the present invention can be used as, for example, a medicament in the regenerative medicine and the regeneration-inducing medicine.

Here, the composition for use in regeneration of tissues as used herein is also expressed as a composition for use in induction or promotion of regeneration of tissues, an agent for use in induction or promotion of regeneration of tissues, a tissue regeneration-inducing agent or a tissue regeneration-promoting agent. In addition, the regeneration of tissues includes tissue repair.

The composition for use in regeneration of tissues of the present invention can be administered or added to any sites. In other words, the composition can exhibit its effects even when administered to any of tissues such as tissues in need of regeneration, tissues different from the tissues in need of regeneration, in blood, or the like. For example, when the composition is administered or added, the cells are recruited to a site to be administered or to be added or a tissue in a vicinity thereof, thereby inducing or promoting the regeneration of tissues. Also, for example, when the composition is administered or added to a damaged tissue site or a vicinity thereof, the cells are recruited in the damaged tissues, thereby inducing or promoting the regeneration of tissues. In addition, for example, when the composition is administered or added to a tissue different from the tissues in need of regeneration, the bone marrow cells are recruited from the bone marrow to the tissues in need of regeneration via the peripheral circulation, thereby inducing or promoting the regeneration of tissues. Here, the "peripheral circulation" is also called "blood circulation" or "peripheral circulation bloodstream."

The tissues in need of regeneration include damaged tissues, necrotic tissues, tissues after surgery, tissues with reduced function, fibrotic tissues, aged tissues, diseased tissues, and the like. Examples include live body skin tissues and tissues from internal biopsy (surgery) (brain, lungs, heart, liver, stomach, small intestine, large intestine, pancreas, kidneys, urinary bladders, spleens, uterus, testis, blood, etc.).

The administration to the tissues different from the tissues in need of regeneration means administration to a site other than a site in need of regeneration (site different from the site in need of regeneration). Therefore, the "tissues different from the tissues in need of regeneration" can be expressed as a site different from the tissues in need of regeneration, a site different from a site in need of regeneration, a site away from the tissues in need of regeneration, a site away from a site in need of regeneration, a site distal from a site in need of regeneration, a site distal from the tissues in need of regeneration, a distal site, or a distal tissue. In other words, the composition of the present invention is effectively used in regeneration of tissues (brain, heart, etc.) that are difficult to be directly administered with a pharmaceutical agent from outside of the body.

The cells recruited to the tissues in need of regeneration differentiate into various cells to contribute to functional regeneration of the tissues in need of regeneration and to maintenance of functions or enhancement of functions of the tissues. In the present invention, the tissues in need of regeneration include tissues damaged by various pathologies caused by ischemic, hypoperfusive or hypoxic conditions, trauma, burns, inflammation, autoimmunity, genetic abnormalities, and the like, without being limited to these causes.

The tissues in the present invention are not particularly limited so long as they are tissues into which the bone marrow-derived cells can differentiate, which include, for example, all the tissues in the live bodies, such as skin tissues, bone tissues, cartilage tissues, muscle tissues, adipose tissues, cardiomuscular tissues, nervous system tissues, pulmonary tissues, digestive tract tissues, hepatic, biliary, and pancreatic tissues, and urogenital organs. In addition, it is made possible to carry out the treatment of inducing functional tissue regeneration in not only a cutaneous disease such as intractable skin ulcers, skin wounds, hydroa/bullous disease, or alopecia, but also in tissues in need of regeneration of cerebral infarction, myocardial infarction, bone fracture, pulmonary infarction, gastric ulcer, enteritis, or the like by the use of the above composition. The animal species to be administered with the above composition is not particularly limited, and includes mammals, birds, fish, and the like. The mammals include human or non-human animals, including, for example, human, mice, rats, monkeys, pigs, dogs, rabbits, hamsters, guinea pigs, horses, sheep, whales, and the like, but not particularly limited thereto.

In addition, the tissues different from the tissues in need of regeneration include blood tissues, muscular tissues, subcutaneous tissues, intradermal tissues, abdominal cavity, and the like.

The neural tissues include central nervous tissues, but not limited thereto. Also, the composition for use in regeneration of neural tissues can be used, for example, in the treatment of cerebral infarction, cerebral hemorrhage, brain contusion or the like, but not limited thereto. In addition, the composition for use in regeneration of bone tissues can be used, for example, in the treatment of bone fracture, but not limited thereto. Also, the composition for use in regeneration of skin tissues can be used, for example, in the treatment of skin ulcers, insufficient suture closure of surgical wounds, burns, cuts, bruises, skin erosion, abrasions, or the like, but not limited thereto.

In addition, in the present invention, the cells that are stimulated to migrate or the cells that are recruited from bone marrow to peripheral blood include undifferentiated cells and cells in different differentiation stages, but not limited thereto. Also, in the present invention, the cells that are stimulated to migrate or the cells that are recruited from bone marrow to peripheral blood include stem cells, non-stem cells, and the like, but not limited thereto. The stem cells include circulatory stem cells or non-circulatory stem cells. The non-circulatory stem cells include tissue stem cells that reside in the tissues. The circulatory stem cells include circulatory stem cells in blood.

Also, the cells that are stimulated to migrate or the cells that are recruited from bone marrow to peripheral blood include bone marrow-derived cells or hematopoietic stem cells, but not limited thereto. The "hematopoietic stem cells" as used herein are stem cells that can differentiate into blood cells such as red blood cells, platelets, mast cells, and dendritic cells, as well as white blood cells such as neutrophils, eosinophils, basophils, lymphocytes, monocytes, and macrophages. The markers thereof have been known to be CD34-positive, CD133-positive in human, CD34-negative, c-Kit-positive, Sca-1-positive, lineage marker-negative in mice. In addition, the hematopoietic stem cells have the feature that when cultured in a culture dish, it is difficult to culture alone, so that it is necessary to be co-cultured with stromal cells.

The term "bone marrow cells" as used herein means cells that are present in the bone marrow, while the term "bone marrow-derived cells" means "bone marrow cells" recruited or taken out from bone marrow to outside the bone marrow. The term "bone marrow cells" includes cells containing tissue progenitor cell population that are present in the bone marrow. In addition, the term "bone marrow-derived cells" may be cells containing mesoangioblasts, or may be cells excluding the mesoangioblasts.

The tissue progenitor cells are defined as undifferentiated cells having a unidirectional differentiability into specified tissue cells other than the blood system, and include undifferentiated cells having differentiability into mesenchymal tissues, epithelial tissues, neural tissues, parenchymatous organs, and vascular endothelia mentioned above.

The terms "bone marrow cells" and "bone marrow-derived cells" are hematopoietic stem cells and differentiated cells derived therefrom such as white blood cells, red blood cells, platelets, osteoblasts, and fibrocytes, or are stem cells which are represented by cells which have been hitherto called bone marrow mesenchymal stem cells, bone marrow mesenchymal stromal cells, bone marrow pluripotent stromal cells, or bone marrow pluripotent stem cells. The term "bone marrow stem cells" as used herein means stem cells existing in the bone marrow, and on the other hand, the term "bone marrow-derived stem cells" means "bone marrow stem cells" that are recruited or taken out from the bone marrow to outside of the bone marrow. In the present invention, the cells that are stimulated to migrate or the cells that are recruited from bone marrow to peripheral blood include "bone marrow-derived stem cells," but not limited thereto. The "bone marrow cells" and the "bone marrow-derived cells" can be isolated by collection from bone marrow (collection of bone marrow cells), or peripheral blood collection. The hematopoietic stem cells are non-adherent cells, and some of the "bone marrow cells" and the "bone marrow-derived cells" are obtained as adherent cells by means of a cell culture of a monocyte fraction in blood obtained by collection from bone marrow (collection of bone marrow cells), or peripheral blood collection.

In addition, the "bone marrow cells" and the "bone marrow-derived cells" include mesenchymal stem cells, and preferably have a potential to differentiate into osteoblasts (which can be specified by observing calcium deposition when inducing differentiation), chondrocytes (which can be specified by Alcian blue staining-positive, safranin-O staining positive, etc.), adipocytes (which can be specified by Sudan III staining positive), and further mesenchymal cells such as fibroblasts, smooth muscle cells, stromal cells, tendon cells, further neural cells, epithelial cells (e.g., epidermal keratinocytes and intestinal epithelial cells expressing cytokeratin family), and vascular endothelial cells. The differentiated cells are not limited to the above cells, and also include the potential to differentiate into cells of parenchymatous organs such as liver, kidneys, and pancreas.

The term "bone marrow stromal cells" as used herein refer to cells other than the hematopoietic stem cells and the blood cells derived therefrom among the cells that exist in the bone marrow, which are collected from the bone marrow and capable of being cultured and proliferated as adherent cells on a culture dish. The bone marrow stromal cells or the bone marrow-derived stromal cells that are taken outside of the bone marrow include cells having pluripotency (bone marrow pluripotent stromal cells or bone marrow-derived pluripotent stromal cells). In addition, the bone marrow-derived stromal cells may be established as a cell line. Examples of the bone marrow-derived stromal cells in cell line include ST2 cells (Ogawa et al., EMBO J 1988; 7:1337-43), and the like. The ST2 cells are bone marrow-derived pluripotent stromal cell lines having a differentiating ability into osteoblasts and adipocytes.

The term "bone marrow mesenchymal stem cells" as used herein is used interchangeably with the terms "bone marrow mesenchymal stromal cells," "bone marrow pluripotent stromal cells," and "bone marrow pluripotent stem cells." The "bone marrow mesenchymal stem cells" as used herein refer to cells that exist in the bone marrow, which are collected directly from the bone marrow, or indirectly from other tissues (blood, skin, adipose, and other tissues), and can be cultured and proliferated as adherent cells on a culture dish (made of plastic or glass). The cells have the characteristics of having a potential to differentiate into mesenchymal tissues such as bones, cartilages, and adipose (mesenchymal stem cells), or into skeletal muscles, heart muscles, and further neural tissues, and epithelial tissues (pluripotent stem cells), and can be obtained by collection of bone marrow cells.

On the other hand, the "bone marrow-derived mesenchymal stem cells," the "bone marrow-derived mesenchymal stromal cells," the "bone marrow-derived pluripotent stromal cells," or the "bone marrow-derived pluripotent stem cells" that are recruited from bone marrow to outside the bone marrow are cells which can be obtained by collection of peripheral blood, or further by collection from mesenchymal tissues such as adipose, epithelial tissues such as skins, or neural tissues such as brain.

In addition, these cells also have the characteristics of having a potential to differentiate into, for example, epithelial tissues such as keratinocytes constituting the skins, or tissues of the nervous system constituting the brain, when the cells are administered to a damaged site of live bodies immediately after collection or after once being adhered onto a culture dish.

The bone marrow mesenchymal stem cells, the bone marrow mesenchymal stromal cells, the bone marrow pluripotent stromal cells, and the bone marrow pluripotent stem cells, or these cells that are recruited from the bone marrow to outside the bone marrow preferably have a potential to differentiate into osteoblasts (which can be specified by observing calcium deposition when inducing differentiation), chondrocytes (which can be specified by Alcian blue staining-positive, safranin-O staining positive, etc.), adipocytes (which can be specified by Sudan III staining positive), and, for example, mesenchymal cells such as fibroblasts, smooth muscle cells, skeletal muscle cells, stromal cells, and tendon cells; neural cells, pigment cells, epidermal cells, hair follicle cells (expressing cytokeratin family, hair keratin family, etc.), epithelial cells (e.g., epidermal keratinocytes and intestinal epithelial cells expressing cytokeratin family), and endothelial cells, and further have a potential to differentiate into cells of parenchymatous organs such as liver, kidneys, and pancreas, but the differentiated cells are not limited to the above cells.

Human bone marrow cells and human bone marrow-derived cells include, but are not limited to, cells that can be obtained by collection from bone marrow (collection of bone marrow cells), peripheral blood collection, or collection of adipose, and can be obtained as adherent cells by direct culture or by culturing an isolated monocyte fraction. Markers for the human bone marrow cells or the human bone marrow-derived cells (e.g., bone marrow mesenchymal stem cells) include all or part(s) of PDGFRα-positive, PDGFRβ-positive, Lin-negative, CD45-negative, CD44-positive, CD90-positive, CD29-positive, Flk-1-negative, CD105-positive, CD73-positive, CD90-positive, CD71-positive, Stro-1-positive, CD106-positive, CD166-positive, CD31-negative, CD271-positive, and CD11b-negative, but not limited thereto.

In addition, murine bone marrow cells and murine bone marrow-derived cells include, but are not limited to, cells that can be obtained by collection from bone marrow (collection of bone marrow cells), peripheral blood collection, or collection of adipose, and can be obtained as adherent cells by direct culture or by culturing an isolated monocyte fraction. Markers for the murine bone marrow cells or murine marrow-derived cells (e.g., bone marrow mesenchymal stem cells) include all or part(s) of CD44-positive, PDGFRα-positive, PDGFRβ-positive, CD45-negative, Lin-negative, Sca-1-positive, c-kit-negative, CD90-positive, CD29-positive, Flk-1-negative, CD271-positive, and CD11b-negative, but not limited thereto.

In the present invention, the cells that are stimulated to migrate or the cells that are recruited from bone marrow to peripheral blood include PDGFRα-positive cells, but not limited thereto. In addition, the markers other than PDGFRα include all or part(s) of CD29-positive, CD44-positive, CD90-positive, CD271-positive, CD11b-negative, and Flk-1-negative, but not limited thereto. The PDGFRα-positive cells include, but are not limited to, PDGFRα-positive bone marrow-derived cells, PDGFRα-positive bone marrow-derived mesenchymal stem cells, tissue cells residing in the PDGFRα-positive tissues (including, e.g., fibroblasts, etc.), PDGFRα-positive bone marrow-derived cells obtained as adherent cells by cell culture of a monocyte fraction in blood obtained by collection from bone marrow (collection of bone marrow cells) or by peripheral blood collection, or the like.

The composition of the present invention may contain a substance other than at least one member of the peptides as defined in any one of the above (a) to (c). In the composition of the present invention, the substance other than at least one member of the peptides as defined in the above (a) to (c) is not particularly limited so long as the substance does not inhibit the activity of stimulating cell migration, the activity of recruiting the cells, or the activity of promoting tissue regeneration. For example, the composition of the present invention may contain, in addition to at least one member of the peptides as defined in the above (a) to (c), related molecule(s) enhancing the function(s) of the peptides as defined in the above (a) to (c), molecule(s) suppressing the actions other than the expected effects of the peptides as defined in the above (a) to (c), factors controlling proliferation or differentiation of cells, and other factors enhancing and maintaining the functions of these factors or cells.

The human HMGB1 protein in the present invention means a protein consisting of the amino acid sequence shown in SEQ ID NO: 401 as a human-derived HMGB 1 protein. In addition, a peptide consisting of an amino acid sequence of position 1 to position 44 of the HMGB1 protein means a peptide consisting of the amino acid sequence shown in SEQ ID NO: 402.

The activity of stimulating cell migration of the peptide can be confirmed by, for example, a method described in Examples, or a method disclosed in WO 2012/147470, but not limited thereto.

The stability of the peptide in plasma can be confirmed by, for example, a method described in Examples, but not limited thereto.

The peptide of the present invention can be exemplified by the peptides consisting of the following amino acid sequences, but not limited to the followings.
(1) MGKGDPKKPR GKMSSYAFFV QTCREEHKKK HPDASVNFSE FSKP (SEQ ID NO: 1)
(2) MGKGDPKKPR GKMSSYAFFV QTCREEHKKK HPDASVNFSE FSPK (SEQ ID NO: 2)
(3) MGKGDPKKPR GKMSSYAFFV QTCREEHKKK HPDASVNFSE FSPP (SEQ ID NO: 3)
(4) MGKGDPKKPR GKMSSYAFFV QTCREEHKKK HPDASVNFSE FSKA (SEQ ID NO: 4)
(5) MGKGDPKKPR GKMSSYAFFV QTCREEHKKK HPDASVNFSE FSKE (SEQ ID NO: 5)
(6) MGKGDPKKPR GKMSSYAFFV QTCREEHKKK HPDASVNFSE FSAA (SEQ ID NO: 6)
(7) MGKGDPKKPR GKMSSYAFFV QTCREEHKKK HPDASVNFSE FSAC (SEQ ID NO: 7)
(8) MGKGDPKKPR GKMSSYAFFV QTCREEHKKK HPDASVNFSE FSAD (SEQ ID NO: 8)
(9) MGKGDPKKPR GKMSSYAFFV QTCREEHKKK HPDASVNFSE FSAE (SEQ ID NO: 9)
(10) MGKGDPKKPR GKMSSYAFFV QTCREEHKKK HPDASVNFSE FSAF (SEQ ID NO: 10)
(11) MGKGDPKKPR GKMSSYAFFV QTCREEHKKK HPDASVNFSE FSAG (SEQ ID NO: 11)
(12) MGKGDPKKPR GKMSSYAFFV QTCREEHKKK HPDASVNFSE FSAH (SEQ ID NO: 12)
(13) MGKGDPKKPR GKMSSYAFFV QTCREEHKKK HPDASVNFSE FSAI (SEQ ID NO: 13)
(14) MGKGDPKKPR GKMSSYAFFV QTCREEHKKK HPDASVNFSE FSAK (SEQ ID NO: 14)
(15) MGKGDPKKPR GKMSSYAFFV QTCREEHKKK HPDASVNFSE FSAL (SEQ ID NO: 15)
(16) MGKGDPKKPR GKMSSYAFFV QTCREEHKKK HPDASVNFSE FSAM (SEQ ID NO: 16)
(17) MGKGDPKKPR GKMSSYAFFV QTCREEHKKK HPDASVNFSE FSAN (SEQ ID NO: 17)
(18) MGKGDPKKPR GKMSSYAFFV QTCREEHKKK HPDASVNFSE FSAP (SEQ ID NO: 18)
(19) MGKGDPKKPR GKMSSYAFFV QTCREEHKKK HPDASVNFSE FSAQ (SEQ ID NO: 19)
(20) MGKGDPKKPR GKMSSYAFFV QTCREEHKKK HPDASVNFSE FSAR (SEQ ID NO: 20)
(21) MGKGDPKKPR GKMSSYAFFV QTCREEHKKK HPDASVNFSE FSAS (SEQ ID NO: 21)
(22) MGKGDPKKPR GKMSSYAFFV QTCREEHKKK HPDASVNFSE FSAT (SEQ ID NO: 22)
(23) MGKGDPKKPR GKMSSYAFFV QTCREEHKKK HPDASVNFSE FSAV (SEQ ID NO: 23)
(24) MGKGDPKKPR GKMSSYAFFV QTCREEHKKK HPDASVNFSE FSAW (SEQ ID NO: 24)
(25) MGKGDPKKPR GKMSSYAFFV QTCREEHKKK HPDASVNFSE FSAY (SEQ ID NO: 25)
(26) MGKGDPKKPR GKMSSYAFFV QTCREEHKKK HPDASVNFSE FSCA (SEQ ID NO: 26)
(27) MGKGDPKKPR GKMSSYAFFV QTCREEHKKK HPDASVNFSE FSCC (SEQ ID NO: 27)
(28) MGKGDPKKPR GKMSSYAFFV QTCREEHKKK HPDASVNFSE FSCD (SEQ ID NO: 28)
(29) MGKGDPKKPR GKMSSYAFFV QTCREEHKKK HPDASVNFSE FSCE (SEQ ID NO: 29)
(30) MGKGDPKKPR GKMSSYAFFV QTCREEHKKK HPDASVNFSE FSCF (SEQ ID NO: 30)
(31) MGKGDPKKPR GKMSSYAFFV QTCREEHKKK HPDASVNFSE FSCG (SEQ ID NO: 31)
(32) MGKGDPKKPR GKMSSYAFFV QTCREEHKKK HPDASVNFSE FSCH (SEQ ID NO: 32)
(33) MGKGDPKKPR GKMSSYAFFV QTCREEHKKK HPDASVNFSE FSCI (SEQ ID NO: 33)
(34) MGKGDPKKPR GKMSSYAFFV QTCREEHKKK HPDASVNFSE FSCK (SEQ ID NO: 34)
(35) MGKGDPKKPR GKMSSYAFFV QTCREEHKKK HPDASVNFSE FSCL (SEQ ID NO: 35)
(36) MGKGDPKKPR GKMSSYAFFV QTCREEHKKK HPDASVNFSE FSCM (SEQ ID NO: 36)
(37) MGKGDPKKPR GKMSSYAFFV QTCREEHKKK HPDASVNFSE FSCN (SEQ ID NO: 37)
(38) MGKGDPKKPR GKMSSYAFFV QTCREEHKKK HPDASVNFSE FSCP (SEQ ID NO: 38)
(39) MGKGDPKKPR GKMSSYAFFV QTCREEHKKK HPDASVNFSE FSCQ (SEQ ID NO: 39)
(40) MGKGDPKKPR GKMSSYAFFV QTCREEHKKK HPDASVNFSE FSCR (SEQ ID NO: 40)
(41) MGKGDPKKPR GKMSSYAFFV QTCREEHKKK HPDASVNFSE FSCS (SEQ ID NO: 41)
(42) MGKGDPKKPR GKMSSYAFFV QTCREEHKKK HPDASVNFSE FSCT (SEQ ID NO: 42)
(43) MGKGDPKKPR GKMSSYAFFV QTCREEHKKK HPDASVNFSE FSCV (SEQ ID NO: 43)
(44) MGKGDPKKPR GKMSSYAFFV QTCREEHKKK HPDASVNFSE FSCW (SEQ ID NO: 44)
(45) MGKGDPKKPR GKMSSYAFFV QTCREEHKKK HPDASVNFSE FSCY (SEQ ID NO: 45)
(46) MGKGDPKKPR GKMSSYAFFV QTCREEHKKK HPDASVNFSE FSDA (SEQ ID NO: 46)
(47) MGKGDPKKPR GKMSSYAFFV QTCREEHKKK HPDASVNFSE FSDC (SEQ ID NO: 47)
(48) MGKGDPKKPR GKMSSYAFFV QTCREEHKKK HPDASVNFSE FSDD (SEQ ID NO: 48)
(49) MGKGDPKKPR GKMSSYAFFV QTCREEHKKK HPDASVNFSE FSDE (SEQ ID NO: 49)
(50) MGKGDPKKPR GKMSSYAFFV QTCREEHKKK HPDASVNFSE FSDF (SEQ ID NO: 50)
(51) MGKGDPKKPR GKMSSYAFFV QTCREEHKKK HPDASVNFSE FSDG (SEQ ID NO: 51)
(52) MGKGDPKKPR GKMSSYAFFV QTCREEHKKK HPDASVNFSE FSDH (SEQ ID NO: 52)
(53) MGKGDPKKPR GKMSSYAFFV QTCREEHKKK HPDASVNFSE FSDI (SEQ ID NO: 53)
(54) MGKGDPKKPR GKMSSYAFFV QTCREEHKKK HPDASVNFSE FSDK (SEQ ID NO: 54)
(55) MGKGDPKKPR GKMSSYAFFV QTCREEHKKK HPDASVNFSE FSDL (SEQ ID NO: 55)
(56) MGKGDPKKPR GKMSSYAFFV QTCREEHKKK HPDASVNFSE FSDM (SEQ ID NO: 56)
(57) MGKGDPKKPR GKMSSYAFFV QTCREEHKKK HPDASVNFSE FSDN (SEQ ID NO: 57)
(58) MGKGDPKKPR GKMSSYAFFV QTCREEHKKK HPDASVNFSE FSDP (SEQ ID NO: 58)
(59) MGKGDPKKPR GKMSSYAFFV QTCREEHKKK HPDASVNFSE FSDQ (SEQ ID NO: 59)
(60) MGKGDPKKPR GKMSSYAFFV QTCREEHKKK HPDASVNFSE FSDR (SEQ ID NO: 60)
(61) MGKGDPKKPR GKMSSYAFFV QTCREEHKKK HPDASVNFSE FSDS (SEQ ID NO: 61)
(62) MGKGDPKKPR GKMSSYAFFV QTCREEHKKK HPDASVNFSE FSDT (SEQ ID NO: 62)
(63) MGKGDPKKPR GKMSSYAFFV QTCREEHKKK HPDASVNFSE FSDV (SEQ ID NO: 63)
(64) MGKGDPKKPR GKMSSYAFFV QTCREEHKKK HPDASVNFSE FSDW (SEQ ID NO: 64)
(65) MGKGDPKKPR GKMSSYAFFV QTCREEHKKK HPDASVNFSE FSDY (SEQ ID NO: 65)
(66) MGKGDPKKPR GKMSSYAFFV QTCREEHKKK HPDASVNFSE FSEA (SEQ ID NO: 66)
(67) MGKGDPKKPR GKMSSYAFFV QTCREEHKKK HPDASVNFSE FSEC (SEQ ID NO: 67)
(68) MGKGDPKKPR GKMSSYAFFV QTCREEHKKK HPDASVNFSE FSED (SEQ ID NO: 68)
(69) MGKGDPKKPR GKMSSYAFFV QTCREEHKKK HPDASVNFSE FSEE (SEQ ID NO: 69)
(70) MGKGDPKKPR GKMSSYAFFV QTCREEHKKK HPDASVNFSE FSEF (SEQ ID NO: 70)
(71) MGKGDPKKPR GKMSSYAFFV QTCREEHKKK HPDASVNFSE FSEG (SEQ ID NO: 71)
(72) MGKGDPKKPR GKMSSYAFFV QTCREEHKKK HPDASVNFSE FSEH (SEQ ID NO: 72)
(73) MGKGDPKKPR GKMSSYAFFV QTCREEHKKK HPDASVNFSE FSEI (SEQ ID NO: 73)
(74) MGKGDPKKPR GKMSSYAFFV QTCREEHKKK HPDASVNFSE FSEK (SEQ ID NO: 74)
(75) MGKGDPKKPR GKMSSYAFFV QTCREEHKKK HPDASVNFSE FSEL (SEQ ID NO: 75)
(76) MGKGDPKKPR GKMSSYAFFV QTCREEHKKK HPDASVNFSE FSEM (SEQ ID NO: 76)
(77) MGKGDPKKPR GKMSSYAFFV QTCREEHKKK HPDASVNFSE FSEN (SEQ ID NO: 77)
(78) MGKGDPKKPR GKMSSYAFFV QTCREEHKKK HPDASVNFSE FSEP (SEQ ID NO: 78)
(79) MGKGDPKKPR GKMSSYAFFV QTCREEHKKK HPDASVNFSE FSEQ (SEQ ID NO: 79)
(80) MGKGDPKKPR GKMSSYAFFV QTCREEHKKK HPDASVNFSE FSER (SEQ ID NO: 80)
(81) MGKGDPKKPR GKMSSYAFFV QTCREEHKKK HPDASVNFSE FSES (SEQ ID NO: 81)
(82) MGKGDPKKPR GKMSSYAFFV QTCREEHKKK HPDASVNFSE FSET (SEQ ID NO: 82)
(83) MGKGDPKKPR GKMSSYAFFV QTCREEHKKK HPDASVNFSE FSEV (SEQ ID NO: 83)
(84) MGKGDPKKPR GKMSSYAFFV QTCREEHKKK HPDASVNFSE FSEW (SEQ ID NO: 84)
(85) MGKGDPKKPR GKMSSYAFFV QTCREEHKKK HPDASVNFSE FSEY (SEQ ID NO: 85)
(86) MGKGDPKKPR GKMSSYAFFV QTCREEHKKK HPDASVNFSE FSFA (SEQ ID NO: 86)
(87) MGKGDPKKPR GKMSSYAFFV QTCREEHKKK HPDASVNFSE FSFC (SEQ ID NO: 87)
(88) MGKGDPKKPR GKMSSYAFFV QTCREEHKKK HPDASVNFSE FSFD (SEQ ID NO: 88)
(89) MGKGDPKKPR GKMSSYAFFV QTCREEHKKK HPDASVNFSE FSFE (SEQ ID NO: 89)
(90) MGKGDPKKPR GKMSSYAFFV QTCREEHKKK HPDASVNFSE FSFF (SEQ ID NO: 90)
(91) MGKGDPKKPR GKMSSYAFFV QTCREEHKKK HPDASVNFSE FSFG (SEQ ID NO: 91)
(92) MGKGDPKKPR GKMSSYAFFV QTCREEHKKK HPDASVNFSE FSFH (SEQ ID NO: 92)
(93) MGKGDPKKPR GKMSSYAFFV QTCREEHKKK HPDASVNFSE FSFI (SEQ ID NO: 93)
(94) MGKGDPKKPR GKMSSYAFFV QTCREEHKKK HPDASVNFSE FSFK (SEQ ID NO: 94)
(95) MGKGDPKKPR GKMSSYAFFV QTCREEHKKK HPDASVNFSE FSFL (SEQ ID NO: 95)
(96) MGKGDPKKPR GKMSSYAFFV QTCREEHKKK HPDASVNFSE FSFM (SEQ ID NO: 96)
(97) MGKGDPKKPR GKMSSYAFFV QTCREEHKKK HPDASVNFSE FSFN (SEQ ID NO: 97)
(98) MGKGDPKKPR GKMSSYAFFV QTCREEHKKK HPDASVNFSE FSFP (SEQ ID NO: 98)
(99) MGKGDPKKPR GKMSSYAFFV QTCREEHKKK HPDASVNFSE FSFQ (SEQ ID NO: 99)
(100) MGKGDPKKPR GKMSSYAFFV QTCREEHKKK HPDASVNFSE FSFR (SEQ ID NO: 100)
(101) MGKGDPKKPR GKMSSYAFFV QTCREEHKKK HPDASVNFSE FSFS (SEQ ID NO: 101)
(102) MGKGDPKKPR GKMSSYAFFV QTCREEHKKK HPDASVNFSE FSFT (SEQ ID NO: 102)
(103) MGKGDPKKPR GKMSSYAFFV QTCREEHKKK HPDASVNFSE FSFV (SEQ ID NO: 103)
(104) MGKGDPKKPR GKMSSYAFFV QTCREEHKKK HPDASVNFSE FSFW (SEQ ID NO: 104)
(105) MGKGDPKKPR GKMSSYAFFV QTCREEHKKK HPDASVNFSE FSFY (SEQ ID NO: 105)
(106) MGKGDPKKPR GKMSSYAFFV QTCREEHKKK HPDASVNFSE FSGA (SEQ ID NO: 106)
(107) MGKGDPKKPR GKMSSYAFFV QTCREEHKKK HPDASVNFSE FSGC (SEQ ID NO: 107)
(108) MGKGDPKKPR GKMSSYAFFV QTCREEHKKK HPDASVNFSE FSGD (SEQ ID NO: 108)
(109) MGKGDPKKPR GKMSSYAFFV QTCREEHKKK HPDASVNFSE FSGE (SEQ ID NO: 109)
(110) MGKGDPKKPR GKMSSYAFFV QTCREEHKKK HPDASVNFSE FSGF (SEQ ID NO: 110)
(111) MGKGDPKKPR GKMSSYAFFV QTCREEHKKK HPDASVNFSE FSGG (SEQ ID NO: 111)
(112) MGKGDPKKPR GKMSSYAFFV QTCREEHKKK HPDASVNFSE FSGH (SEQ ID NO: 112)
(113) MGKGDPKKPR GKMSSYAFFV QTCREEHKKK HPDASVNFSE FSGI (SEQ ID NO: 113)
(114) MGKGDPKKPR GKMSSYAFFV QTCREEHKKK HPDASVNFSE FSGK (SEQ ID NO: 114)
(115) MGKGDPKKPR GKMSSYAFFV QTCREEHKKK HPDASVNFSE FSGL (SEQ ID NO: 115)
(116) MGKGDPKKPR GKMSSYAFFV QTCREEHKKK HPDASVNFSE FSGM (SEQ ID NO: 116)
(117) MGKGDPKKPR GKMSSYAFFV QTCREEHKKK HPDASVNFSE FSGN (SEQ ID NO: 117)
(118) MGKGDPKKPR GKMSSYAFFV QTCREEHKKK HPDASVNFSE FSGP (SEQ ID NO: 118)
(119) MGKGDPKKPR GKMSSYAFFV QTCREEHKKK HPDASVNFSE FSGQ (SEQ ID NO: 119)
(120) MGKGDPKKPR GKMSSYAFFV QTCREEHKKK HPDASVNFSE FSGR (SEQ ID NO: 120)
(121) MGKGDPKKPR GKMSSYAFFV QTCREEHKKK HPDASVNFSE FSGS (SEQ ID NO: 121)
(122) MGKGDPKKPR GKMSSYAFFV QTCREEHKKK HPDASVNFSE FSGT (SEQ ID NO: 122)
(123) MGKGDPKKPR GKMSSYAFFV QTCREEHKKK HPDASVNFSE FSGV (SEQ ID NO: 123)
(124) MGKGDPKKPR GKMSSYAFFV QTCREEHKKK HPDASVNFSE FSGW (SEQ ID NO: 124)
(125) MGKGDPKKPR GKMSSYAFFV QTCREEHKKK HPDASVNFSE FSGY (SEQ ID NO: 125)
(126) MGKGDPKKPR GKMSSYAFFV QTCREEHKKK HPDASVNFSE FSHA (SEQ ID NO: 126)
(127) MGKGDPKKPR GKMSSYAFFV QTCREEHKKK HPDASVNFSE FSHC (SEQ ID NO: 127)
(128) MGKGDPKKPR GKMSSYAFFV QTCREEHKKK HPDASVNFSE FSHD (SEQ ID NO: 128)
(129) MGKGDPKKPR GKMSSYAFFV QTCREEHKKK HPDASVNFSE FSHE (SEQ ID NO: 129)
(130) MGKGDPKKPR GKMSSYAFFV QTCREEHKKK HPDASVNFSE FSHF (SEQ ID NO: 130)
(131) MGKGDPKKPR GKMSSYAFFV QTCREEHKKK HPDASVNFSE FSHG (SEQ ID NO: 131)
(132) MGKGDPKKPR GKMSSYAFFV QTCREEHKKK HPDASVNFSE FSHH (SEQ ID NO: 132)
(133) MGKGDPKKPR GKMSSYAFFV QTCREEHKKK HPDASVNFSE FSHI (SEQ ID NO: 133)
(134) MGKGDPKKPR GKMSSYAFFV QTCREEHKKK HPDASVNFSE FSHK (SEQ ID NO: 134)
(135) MGKGDPKKPR GKMSSYAFFV QTCREEHKKK HPDASVNFSE FSHL (SEQ ID NO: 135)
(136) MGKGDPKKPR GKMSSYAFFV QTCREEHKKK HPDASVNFSE FSHM (SEQ ID NO: 136)
(137) MGKGDPKKPR GKMSSYAFFV QTCREEHKKK HPDASVNFSE FSHN (SEQ ID NO: 137)
(138) MGKGDPKKPR GKMSSYAFFV QTCREEHKKK HPDASVNFSE FSHP (SEQ ID NO: 138)
(139) MGKGDPKKPR GKMSSYAFFV QTCREEHKKK HPDASVNFSE FSHQ (SEQ ID NO: 139)
(140) MGKGDPKKPR GKMSSYAFFV QTCREEHKKK HPDASVNFSE FSHR (SEQ ID NO: 140)
(141) MGKGDPKKPR GKMSSYAFFV QTCREEHKKK HPDASVNFSE FSHS (SEQ ID NO: 141)
(142) MGKGDPKKPR GKMSSYAFFV QTCREEHKKK HPDASVNFSE FSHT (SEQ ID NO: 142)
(143) MGKGDPKKPR GKMSSYAFFV QTCREEHKKK HPDASVNFSE FSHV (SEQ ID NO: 143)
(144) MGKGDPKKPR GKMSSYAFFV QTCREEHKKK HPDASVNFSE FSHW (SEQ ID NO: 144)
(145) MGKGDPKKPR GKMSSYAFFV QTCREEHKKK HPDASVNFSE FSHY (SEQ ID NO: 145)
(146) MGKGDPKKPR GKMSSYAFFV QTCREEHKKK HPDASVNFSE FSIA (SEQ ID NO: 146)
(147) MGKGDPKKPR GKMSSYAFFV QTCREEHKKK HPDASVNFSE FSIC (SEQ ID NO: 147)
(148) MGKGDPKKPR GKMSSYAFFV QTCREEHKKK HPDASVNFSE FSID (SEQ ID NO: 148)
(149) MGKGDPKKPR GKMSSYAFFV QTCREEHKKK HPDASVNFSE FSIE (SEQ ID NO: 149)
(150) MGKGDPKKPR GKMSSYAFFV QTCREEHKKK HPDASVNFSE FSIF (SEQ ID NO: 150)
(151) MGKGDPKKPR GKMSSYAFFV QTCREEHKKK HPDASVNFSE FSIG (SEQ ID NO: 151)
(152) MGKGDPKKPR GKMSSYAFFV QTCREEHKKK HPDASVNFSE FSIH (SEQ ID NO: 152)
(153) MGKGDPKKPR GKMSSYAFFV QTCREEHKKK HPDASVNFSE FSII (SEQ ID NO: 153)
(154) MGKGDPKKPR GKMSSYAFFV QTCREEHKKK HPDASVNFSE FSIK (SEQ ID NO: 154)
(155) MGKGDPKKPR GKMSSYAFFV QTCREEHKKK HPDASVNFSE FSIL (SEQ ID NO: 155)
(156) MGKGDPKKPR GKMSSYAFFV QTCREEHKKK HPDASVNFSE FSIM (SEQ ID NO: 156)
(157) MGKGDPKKPR GKMSSYAFFV QTCREEHKKK HPDASVNFSE FSIN (SEQ ID NO: 157)
(158) MGKGDPKKPR GKMSSYAFFV QTCREEHKKK HPDASVNFSE FSIP (SEQ ID NO: 158)
(159) MGKGDPKKPR GKMSSYAFFV QTCREEHKKK HPDASVNFSE FSIQ (SEQ ID NO: 159)
(160) MGKGDPKKPR GKMSSYAFFV QTCREEHKKK HPDASVNFSE FSIR (SEQ ID NO: 160)
(161) MGKGDPKKPR GKMSSYAFFV QTCREEHKKK HPDASVNFSE FSIS (SEQ ID NO: 161)
(162) MGKGDPKKPR GKMSSYAFFV QTCREEHKKK HPDASVNFSE FSIT (SEQ ID NO: 162)
(163) MGKGDPKKPR GKMSSYAFFV QTCREEHKKK HPDASVNFSE FSIV (SEQ ID NO: 163)
(164) MGKGDPKKPR GKMSSYAFFV QTCREEHKKK HPDASVNFSE FSIW (SEQ ID NO: 164)
(165) MGKGDPKKPR GKMSSYAFFV QTCREEHKKK HPDASVNFSE FSIY (SEQ ID NO: 165)
(166) MGKGDPKKPR GKMSSYAFFV QTCREEHKKK HPDASVNFSE FSKC (SEQ ID NO: 166)
(167) MGKGDPKKPR GKMSSYAFFV QTCREEHKKK HPDASVNFSE FSKD (SEQ ID NO: 167)
(168) MGKGDPKKPR GKMSSYAFFV QTCREEHKKK HPDASVNFSE FSKF (SEQ ID NO: 168)
(169) MGKGDPKKPR GKMSSYAFFV QTCREEHKKK HPDASVNFSE FSKG (SEQ ID NO: 169)
(170) MGKGDPKKPR GKMSSYAFFV QTCREEHKKK HPDASVNFSE FSKH (SEQ ID NO: 170)
(171) MGKGDPKKPR GKMSSYAFFV QTCREEHKKK HPDASVNFSE FSKI (SEQ ID NO: 171)
(172) MGKGDPKKPR GKMSSYAFFV QTCREEHKKK HPDASVNFSE FSKL (SEQ ID NO: 172)
(173) MGKGDPKKPR GKMSSYAFFV QTCREEHKKK HPDASVNFSE FSKM (SEQ ID NO: 173)
(174) MGKGDPKKPR GKMSSYAFFV QTCREEHKKK HPDASVNFSE FSKN (SEQ ID NO: 174)
(175) MGKGDPKKPR GKMSSYAFFV QTCREEHKKK HPDASVNFSE FSKQ (SEQ ID NO: 175)
(176) MGKGDPKKPR GKMSSYAFFV QTCREEHKKK HPDASVNFSE FSKR (SEQ ID NO: 176)
(177) MGKGDPKKPR GKMSSYAFFV QTCREEHKKK HPDASVNFSE FSKS (SEQ ID NO: 177)
(178) MGKGDPKKPR GKMSSYAFFV QTCREEHKKK HPDASVNFSE FSKT (SEQ ID NO: 178)
(179) MGKGDPKKPR GKMSSYAFFV QTCREEHKKK HPDASVNFSE FSKV (SEQ ID NO: 179)
(180) MGKGDPKKPR GKMSSYAFFV QTCREEHKKK HPDASVNFSE FSKW (SEQ ID NO: 180)
(181) MGKGDPKKPR GKMSSYAFFV QTCREEHKKK HPDASVNFSE FSKY (SEQ ID NO: 181)
(182) MGKGDPKKPR GKMSSYAFFV QTCREEHKKK HPDASVNFSE FSLA (SEQ ID NO: 182)
(183) MGKGDPKKPR GKMSSYAFFV QTCREEHKKK HPDASVNFSE FSLC (SEQ ID NO: 183)
(184) MGKGDPKKPR GKMSSYAFFV QTCREEHKKK HPDASVNFSE FSLD (SEQ ID NO: 184)
(185) MGKGDPKKPR GKMSSYAFFV QTCREEHKKK HPDASVNFSE FSLE (SEQ ID NO: 185)
(186) MGKGDPKKPR GKMSSYAFFV QTCREEHKKK HPDASVNFSE FSLF (SEQ ID NO: 186)
(187) MGKGDPKKPR GKMSSYAFFV QTCREEHKKK HPDASVNFSE FSLG (SEQ ID NO: 187)
(188) MGKGDPKKPR GKMSSYAFFV QTCREEHKKK HPDASVNFSE FSLH (SEQ ID NO: 188)
(189) MGKGDPKKPR GKMSSYAFFV QTCREEHKKK HPDASVNFSE FSLI (SEQ ID NO: 189)
(190) MGKGDPKKPR GKMSSYAFFV QTCREEHKKK HPDASVNFSE FSLK (SEQ ID NO: 190)
(191) MGKGDPKKPR GKMSSYAFFV QTCREEHKKK HPDASVNFSE FSLL (SEQ ID NO: 191)
(192) MGKGDPKKPR GKMSSYAFFV QTCREEHKKK HPDASVNFSE FSLM (SEQ ID NO: 192)
(193) MGKGDPKKPR GKMSSYAFFV QTCREEHKKK HPDASVNFSE FSLN (SEQ ID NO: 193)
(194) MGKGDPKKPR GKMSSYAFFV QTCREEHKKK HPDASVNFSE FSLP (SEQ ID NO: 194)
(195) MGKGDPKKPR GKMSSYAFFV QTCREEHKKK HPDASVNFSE FSLQ (SEQ ID NO: 195)
(196) MGKGDPKKPR GKMSSYAFFV QTCREEHKKK HPDASVNFSE FSLR (SEQ ID NO: 196)
(197) MGKGDPKKPR GKMSSYAFFV QTCREEHKKK HPDASVNFSE FSLS (SEQ ID NO: 197)
(198) MGKGDPKKPR GKMSSYAFFV QTCREEHKKK HPDASVNFSE FSLT (SEQ ID NO: 198)
(199) MGKGDPKKPR GKMSSYAFFV QTCREEHKKK HPDASVNFSE FSLV (SEQ ID NO: 199)
(200) MGKGDPKKPR GKMSSYAFFV QTCREEHKKK HPDASVNFSE FSLW (SEQ ID NO: 200)
(201) MGKGDPKKPR GKMSSYAFFV QTCREEHKKK HPDASVNFSE FSLY (SEQ ID NO: 201)
(202) MGKGDPKKPR GKMSSYAFFV QTCREEHKKK HPDASVNFSE FSMA (SEQ ID NO: 202)
(203) MGKGDPKKPR GKMSSYAFFV QTCREEHKKK HPDASVNFSE FSMC (SEQ ID NO: 203)
(204) MGKGDPKKPR GKMSSYAFFV QTCREEHKKK HPDASVNFSE FSMD (SEQ ID NO: 204)
(205) MGKGDPKKPR GKMSSYAFFV QTCREEHKKK HPDASVNFSE FSME (SEQ ID NO: 205)
(206) MGKGDPKKPR GKMSSYAFFV QTCREEHKKK HPDASVNFSE FSMF (SEQ ID NO: 206)
(207) MGKGDPKKPR GKMSSYAFFV QTCREEHKKK HPDASVNFSE FSMG (SEQ ID NO: 207)
(208) MGKGDPKKPR GKMSSYAFFV QTCREEHKKK HPDASVNFSE FSMH (SEQ ID NO: 208)
(209) MGKGDPKKPR GKMSSYAFFV QTCREEHKKK HPDASVNFSE FSMI (SEQ ID NO: 209)
(210) MGKGDPKKPR GKMSSYAFFV QTCREEHKKK HPDASVNFSE FSMK (SEQ ID NO: 210)
(211) MGKGDPKKPR GKMSSYAFFV QTCREEHKKK HPDASVNFSE FSML (SEQ ID NO: 211)
(212) MGKGDPKKPR GKMSSYAFFV QTCREEHKKK HPDASVNFSE FSMM (SEQ ID NO: 212)
(213) MGKGDPKKPR GKMSSYAFFV QTCREEHKKK HPDASVNFSE FSMN (SEQ ID NO: 213)
(214) MGKGDPKKPR GKMSSYAFFV QTCREEHKKK HPDASVNFSE FSMP (SEQ ID NO: 214)
(215) MGKGDPKKPR GKMSSYAFFV QTCREEHKKK HPDASVNFSE FSMQ (SEQ ID NO: 215)
(216) MGKGDPKKPR GKMSSYAFFV QTCREEHKKK HPDASVNFSE FSMR (SEQ ID NO: 216)
(217) MGKGDPKKPR GKMSSYAFFV QTCREEHKKK HPDASVNFSE FSMS (SEQ ID NO: 217)
(218) MGKGDPKKPR GKMSSYAFFV QTCREEHKKK HPDASVNFSE FSMT (SEQ ID NO: 218)
(219) MGKGDPKKPR GKMSSYAFFV QTCREEHKKK HPDASVNFSE FSMV (SEQ ID NO: 219)
(220) MGKGDPKKPR GKMSSYAFFV QTCREEHKKK HPDASVNFSE FSMW (SEQ ID NO: 220)
(221) MGKGDPKKPR GKMSSYAFFV QTCREEHKKK HPDASVNFSE FSMY (SEQ ID NO: 221)
(222) MGKGDPKKPR GKMSSYAFFV QTCREEHKKK HPDASVNFSE FSNA (SEQ ID NO: 222)
(223) MGKGDPKKPR GKMSSYAFFV QTCREEHKKK HPDASVNFSE FSNC (SEQ ID NO: 223)
(224) MGKGDPKKPR GKMSSYAFFV QTCREEHKKK HPDASVNFSE FSND (SEQ ID NO: 224)
(225) MGKGDPKKPR GKMSSYAFFV QTCREEHKKK HPDASVNFSE FSNE (SEQ ID NO: 225)
(226) MGKGDPKKPR GKMSSYAFFV QTCREEHKKK HPDASVNFSE FSNF (SEQ ID NO: 226)
(227) MGKGDPKKPR GKMSSYAFFV QTCREEHKKK HPDASVNFSE FSNG (SEQ ID NO: 227)
(228) MGKGDPKKPR GKMSSYAFFV QTCREEHKKK HPDASVNFSE FSNH (SEQ ID NO: 228)
(229) MGKGDPKKPR GKMSSYAFFV QTCREEHKKK HPDASVNFSE FSNI (SEQ ID NO: 229)
(230) MGKGDPKKPR GKMSSYAFFV QTCREEHKKK HPDASVNFSE FSNK (SEQ ID NO: 230)
(231) MGKGDPKKPR GKMSSYAFFV QTCREEHKKK HPDASVNFSE FSNL (SEQ ID NO: 231)
(232) MGKGDPKKPR GKMSSYAFFV QTCREEHKKK HPDASVNFSE FSNM (SEQ ID NO: 232)
(233) MGKGDPKKPR GKMSSYAFFV QTCREEHKKK HPDASVNFSE FSNN (SEQ ID NO: 233)
(234) MGKGDPKKPR GKMSSYAFFV QTCREEHKKK HPDASVNFSE FSNP (SEQ ID NO: 234)
(235) MGKGDPKKPR GKMSSYAFFV QTCREEHKKK HPDASVNFSE FSNQ (SEQ ID NO: 235)
(236) MGKGDPKKPR GKMSSYAFFV QTCREEHKKK HPDASVNFSE FSNR (SEQ ID NO: 236)
(237) MGKGDPKKPR GKMSSYAFFV QTCREEHKKK HPDASVNFSE FSNS (SEQ ID NO: 237)
(238) MGKGDPKKPR GKMSSYAFFV QTCREEHKKK HPDASVNFSE FSNT (SEQ ID NO: 238)
(239) MGKGDPKKPR GKMSSYAFFV QTCREEHKKK HPDASVNFSE FSNV (SEQ ID NO: 239)
(240) MGKGDPKKPR GKMSSYAFFV QTCREEHKKK HPDASVNFSE FSNW (SEQ ID NO: 240)
(241) MGKGDPKKPR GKMSSYAFFV QTCREEHKKK HPDASVNFSE FSNY (SEQ ID NO: 241)
(242) MGKGDPKKPR GKMSSYAFFV QTCREEHKKK HPDASVNFSE FSPA (SEQ ID NO: 242)
(243) MGKGDPKKPR GKMSSYAFFV QTCREEHKKK HPDASVNFSE FSPC (SEQ ID NO: 243)
(244) MGKGDPKKPR GKMSSYAFFV QTCREEHKKK HPDASVNFSE FSPD (SEQ ID NO: 244)
(245) MGKGDPKKPR GKMSSYAFFV QTCREEHKKK HPDASVNFSE FSPE (SEQ ID NO: 245)
(246) MGKGDPKKPR GKMSSYAFFV QTCREEHKKK HPDASVNFSE FSPF (SEQ ID NO: 246)
(247) MGKGDPKKPR GKMSSYAFFV QTCREEHKKK HPDASVNFSE FSPG (SEQ ID NO: 247)
(248) MGKGDPKKPR GKMSSYAFFV QTCREEHKKK HPDASVNFSE FSPH (SEQ ID NO: 248)
(249) MGKGDPKKPR GKMSSYAFFV QTCREEHKKK HPDASVNFSE FSPI (SEQ ID NO: 249)
(250) MGKGDPKKPR GKMSSYAFFV QTCREEHKKK HPDASVNFSE FSPL (SEQ ID NO: 250)
(251) MGKGDPKKPR GKMSSYAFFV QTCREEHKKK HPDASVNFSE FSPM (SEQ ID NO: 251)
(252) MGKGDPKKPR GKMSSYAFFV QTCREEHKKK HPDASVNFSE FSPN (SEQ ID NO: 252)
(253) MGKGDPKKPR GKMSSYAFFV QTCREEHKKK HPDASVNFSE FSPQ (SEQ ID NO: 253)
(254) MGKGDPKKPR GKMSSYAFFV QTCREEHKKK HPDASVNFSE FSPR (SEQ ID NO: 254)
(255) MGKGDPKKPR GKMSSYAFFV QTCREEHKKK HPDASVNFSE FSPS (SEQ ID NO: 255)
(256) MGKGDPKKPR GKMSSYAFFV QTCREEHKKK HPDASVNFSE FSPT (SEQ ID NO: 256)
(257) MGKGDPKKPR GKMSSYAFFV QTCREEHKKK HPDASVNFSE FSPV (SEQ ID NO: 257)
(258) MGKGDPKKPR GKMSSYAFFV QTCREEHKKK HPDASVNFSE FSPW (SEQ ID NO: 258)
(259) MGKGDPKKPR GKMSSYAFFV QTCREEHKKK HPDASVNFSE FSPY (SEQ ID NO: 259)
(260) MGKGDPKKPR GKMSSYAFFV QTCREEHKKK HPDASVNFSE FSQA (SEQ ID NO: 260)
(261) MGKGDPKKPR GKMSSYAFFV QTCREEHKKK HPDASVNFSE FSQC (SEQ ID NO: 261)
(262) MGKGDPKKPR GKMSSYAFFV QTCREEHKKK HPDASVNFSE FSQD (SEQ ID NO: 262)
(263) MGKGDPKKPR GKMSSYAFFV QTCREEHKKK HPDASVNFSE FSQE (SEQ ID NO: 263)
(264) MGKGDPKKPR GKMSSYAFFV QTCREEHKKK HPDASVNFSE FSQF (SEQ ID NO: 264)
(265) MGKGDPKKPR GKMSSYAFFV QTCREEHKKK HPDASVNFSE FSQG (SEQ ID NO: 265)
(266) MGKGDPKKPR GKMSSYAFFV QTCREEHKKK HPDASVNFSE FSQH (SEQ ID NO: 266)
(267) MGKGDPKKPR GKMSSYAFFV QTCREEHKKK HPDASVNFSE FSQI (SEQ ID NO: 267)
(268) MGKGDPKKPR GKMSSYAFFV QTCREEHKKK HPDASVNFSE FSQK (SEQ ID NO: 268)
(269) MGKGDPKKPR GKMSSYAFFV QTCREEHKKK HPDASVNFSE FSQL (SEQ ID NO: 269)
(270) MGKGDPKKPR GKMSSYAFFV QTCREEHKKK HPDASVNFSE FSQM (SEQ ID NO: 270)
(271) MGKGDPKKPR GKMSSYAFFV QTCREEHKKK HPDASVNFSE FSQN (SEQ ID NO: 271)
(272) MGKGDPKKPR GKMSSYAFFV QTCREEHKKK HPDASVNFSE FSQP (SEQ ID NO: 272)
(273) MGKGDPKKPR GKMSSYAFFV QTCREEHKKK HPDASVNFSE FSQQ (SEQ ID NO: 273)
(274) MGKGDPKKPR GKMSSYAFFV QTCREEHKKK HPDASVNFSE FSQR (SEQ ID NO: 274)
(275) MGKGDPKKPR GKMSSYAFFV QTCREEHKKK HPDASVNFSE FSQS (SEQ ID NO: 275)
(276) MGKGDPKKPR GKMSSYAFFV QTCREEHKKK HPDASVNFSE FSQT (SEQ ID NO: 276)
(277) MGKGDPKKPR GKMSSYAFFV QTCREEHKKK HPDASVNFSE FSQV (SEQ ID NO: 277)
(278) MGKGDPKKPR GKMSSYAFFV QTCREEHKKK HPDASVNFSE FSQW (SEQ ID NO: 278)
(279) MGKGDPKKPR GKMSSYAFFV QTCREEHKKK HPDASVNFSE FSQY (SEQ ID NO: 279)
(280) MGKGDPKKPR GKMSSYAFFV QTCREEHKKK HPDASVNFSE FSRA (SEQ ID NO: 280)
(281) MGKGDPKKPR GKMSSYAFFV QTCREEHKKK HPDASVNFSE FSRC (SEQ ID NO: 281)
(282) MGKGDPKKPR GKMSSYAFFV QTCREEHKKK HPDASVNFSE FSRD (SEQ ID NO: 282)
(283) MGKGDPKKPR GKMSSYAFFV QTCREEHKKK HPDASVNFSE FSRE (SEQ ID NO: 283)
(284) MGKGDPKKPR GKMSSYAFFV QTCREEHKKK HPDASVNFSE FSRF (SEQ ID NO: 284)
(285) MGKGDPKKPR GKMSSYAFFV QTCREEHKKK HPDASVNFSE FSRG (SEQ ID NO: 285)
(286) MGKGDPKKPR GKMSSYAFFV QTCREEHKKK HPDASVNFSE FSRH (SEQ ID NO: 286)
(287) MGKGDPKKPR GKMSSYAFFV QTCREEHKKK HPDASVNFSE FSRI (SEQ ID NO: 287)
(288) MGKGDPKKPR GKMSSYAFFV QTCREEHKKK HPDASVNFSE FSRK (SEQ ID NO: 288)
(289) MGKGDPKKPR GKMSSYAFFV QTCREEHKKK HPDASVNFSE FSRL (SEQ ID NO: 289)
(290) MGKGDPKKPR GKMSSYAFFV QTCREEHKKK HPDASVNFSE FSRM (SEQ ID NO: 290)
(291) MGKGDPKKPR GKMSSYAFFV QTCREEHKKK HPDASVNFSE FSRN (SEQ ID NO: 291)
(292) MGKGDPKKPR GKMSSYAFFV QTCREEHKKK HPDASVNFSE FSRP (SEQ ID NO: 292)
(293) MGKGDPKKPR GKMSSYAFFV QTCREEHKKK HPDASVNFSE FSRQ (SEQ ID NO: 293)
(294) MGKGDPKKPR GKMSSYAFFV QTCREEHKKK HPDASVNFSE FSRR (SEQ ID NO: 294)
(295) MGKGDPKKPR GKMSSYAFFV QTCREEHKKK HPDASVNFSE FSRS (SEQ ID NO: 295)
(296) MGKGDPKKPR GKMSSYAFFV QTCREEHKKK HPDASVNFSE FSRT (SEQ ID NO: 296)
(297) MGKGDPKKPR GKMSSYAFFV QTCREEHKKK HPDASVNFSE FSRV (SEQ ID NO: 297)
(298) MGKGDPKKPR GKMSSYAFFV QTCREEHKKK HPDASVNFSE FSRW (SEQ ID NO: 298)
(299) MGKGDPKKPR GKMSSYAFFV QTCREEHKKK HPDASVNFSE FSRY (SEQ ID NO: 299)
(300) MGKGDPKKPR GKMSSYAFFV QTCREEHKKK HPDASVNFSE FSSA (SEQ ID NO: 300)
(301) MGKGDPKKPR GKMSSYAFFV QTCREEHKKK HPDASVNFSE FSSC (SEQ ID NO: 301)
(302) MGKGDPKKPR GKMSSYAFFV QTCREEHKKK HPDASVNFSE FSSD (SEQ ID NO: 302)
(303) MGKGDPKKPR GKMSSYAFFV QTCREEHKKK HPDASVNFSE FSSE (SEQ ID NO: 303)
(304) MGKGDPKKPR GKMSSYAFFV QTCREEHKKK HPDASVNFSE FSSF (SEQ ID NO: 304)
(305) MGKGDPKKPR GKMSSYAFFV QTCREEHKKK HPDASVNFSE FSSG (SEQ ID NO: 305)
(306) MGKGDPKKPR GKMSSYAFFV QTCREEHKKK HPDASVNFSE FSSH (SEQ ID NO: 306)
(307) MGKGDPKKPR GKMSSYAFFV QTCREEHKKK HPDASVNFSE FSSI (SEQ ID NO: 307)
(308) MGKGDPKKPR GKMSSYAFFV QTCREEHKKK HPDASVNFSE FSSK (SEQ ID NO: 308)
(309) MGKGDPKKPR GKMSSYAFFV QTCREEHKKK HPDASVNFSE FSSL (SEQ ID NO: 309)
(310) MGKGDPKKPR GKMSSYAFFV QTCREEHKKK HPDASVNFSE FSSM (SEQ ID NO: 310)
(311) MGKGDPKKPR GKMSSYAFFV QTCREEHKKK HPDASVNFSE FSSN (SEQ ID NO: 311)
(312) MGKGDPKKPR GKMSSYAFFV QTCREEHKKK HPDASVNFSE FSSP (SEQ ID NO: 312)
(313) MGKGDPKKPR GKMSSYAFFV QTCREEHKKK HPDASVNFSE FSSQ (SEQ ID NO: 313)
(314) MGKGDPKKPR GKMSSYAFFV QTCREEHKKK HPDASVNFSE FSSR (SEQ ID NO: 314)
(315) MGKGDPKKPR GKMSSYAFFV QTCREEHKKK HPDASVNFSE FSSS (SEQ ID NO: 315)
(316) MGKGDPKKPR GKMSSYAFFV QTCREEHKKK HPDASVNFSE FSST (SEQ ID NO: 316)
(317) MGKGDPKKPR GKMSSYAFFV QTCREEHKKK HPDASVNFSE FSSV (SEQ ID NO: 317)
(318) MGKGDPKKPR GKMSSYAFFV QTCREEHKKK HPDASVNFSE FSSW (SEQ ID NO: 318)
(319) MGKGDPKKPR GKMSSYAFFV QTCREEHKKK HPDASVNFSE FSSY (SEQ ID NO: 319)
(320) MGKGDPKKPR GKMSSYAFFV QTCREEHKKK HPDASVNFSE FSTA (SEQ ID NO: 320)
(321) MGKGDPKKPR GKMSSYAFFV QTCREEHKKK HPDASVNFSE FSTC (SEQ ID NO: 321)
(322) MGKGDPKKPR GKMSSYAFFV QTCREEHKKK HPDASVNFSE FSTD (SEQ ID NO: 322)
(323) MGKGDPKKPR GKMSSYAFFV QTCREEHKKK HPDASVNFSE FSTE (SEQ ID NO: 323)
(324) MGKGDPKKPR GKMSSYAFFV QTCREEHKKK HPDASVNFSE FSTF (SEQ ID NO: 324)
(325) MGKGDPKKPR GKMSSYAFFV QTCREEHKKK HPDASVNFSE FSTG (SEQ ID NO: 325)
(326) MGKGDPKKPR GKMSSYAFFV QTCREEHKKK HPDASVNFSE FSTH (SEQ ID NO: 326)
(327) MGKGDPKKPR GKMSSYAFFV QTCREEHKKK HPDASVNFSE FSTI (SEQ ID NO: 327)
(328) MGKGDPKKPR GKMSSYAFFV QTCREEHKKK HPDASVNFSE FSTK (SEQ ID NO: 328)
(329) MGKGDPKKPR GKMSSYAFFV QTCREEHKKK HPDASVNFSE FSTL (SEQ ID NO: 329)
(330) MGKGDPKKPR GKMSSYAFFV QTCREEHKKK HPDASVNFSE FSTM (SEQ ID NO: 330)
(331) MGKGDPKKPR GKMSSYAFFV QTCREEHKKK HPDASVNFSE FSTN (SEQ ID NO: 331)
(332) MGKGDPKKPR GKMSSYAFFV QTCREEHKKK HPDASVNFSE FSTP (SEQ ID NO: 332)
(333) MGKGDPKKPR GKMSSYAFFV QTCREEHKKK HPDASVNFSE FSTQ (SEQ ID NO: 333)
(334) MGKGDPKKPR GKMSSYAFFV QTCREEHKKK HPDASVNFSE FSTR (SEQ ID NO: 334)
(335) MGKGDPKKPR GKMSSYAFFV QTCREEHKKK HPDASVNFSE FSTS (SEQ ID NO: 335)
(336) MGKGDPKKPR GKMSSYAFFV QTCREEHKKK HPDASVNFSE FSTT (SEQ ID NO: 336)
(337) MGKGDPKKPR GKMSSYAFFV QTCREEHKKK HPDASVNFSE FSTV (SEQ ID NO: 337)
(338) MGKGDPKKPR GKMSSYAFFV QTCREEHKKK HPDASVNFSE FSTW (SEQ ID NO: 338)
(339) MGKGDPKKPR GKMSSYAFFV QTCREEHKKK HPDASVNFSE FSTY (SEQ ID NO: 339)
(340) MGKGDPKKPR GKMSSYAFFV QTCREEHKKK HPDASVNFSE FSVA (SEQ ID NO: 340)
(341) MGKGDPKKPR GKMSSYAFFV QTCREEHKKK HPDASVNFSE FSVC (SEQ ID NO: 341)
(342) MGKGDPKKPR GKMSSYAFFV QTCREEHKKK HPDASVNFSE FSVD (SEQ ID NO: 342)
(343) MGKGDPKKPR GKMSSYAFFV QTCREEHKKK HPDASVNFSE FSVE (SEQ ID NO: 343)
(344) MGKGDPKKPR GKMSSYAFFV QTCREEHKKK HPDASVNFSE FSVF (SEQ ID NO: 344)
(345) MGKGDPKKPR GKMSSYAFFV QTCREEHKKK HPDASVNFSE FSVG (SEQ ID NO: 345)
(346) MGKGDPKKPR GKMSSYAFFV QTCREEHKKK HPDASVNFSE FSVH (SEQ ID NO: 346)
(347) MGKGDPKKPR GKMSSYAFFV QTCREEHKKK HPDASVNFSE FSVI (SEQ ID NO: 347)
(348) MGKGDPKKPR GKMSSYAFFV QTCREEHKKK HPDASVNFSE FSVK (SEQ ID NO: 348)
(349) MGKGDPKKPR GKMSSYAFFV QTCREEHKKK HPDASVNFSE FSVL (SEQ ID NO: 349)
(350) MGKGDPKKPR GKMSSYAFFV QTCREEHKKK HPDASVNFSE FSVM (SEQ ID NO: 350)
(351) MGKGDPKKPR GKMSSYAFFV QTCREEHKKK HPDASVNFSE FSVN (SEQ ID NO: 351)
(352) MGKGDPKKPR GKMSSYAFFV QTCREEHKKK HPDASVNFSE FSVP (SEQ ID NO: 352)
(353) MGKGDPKKPR GKMSSYAFFV QTCREEHKKK HPDASVNFSE FSVQ (SEQ ID NO: 353)
(354) MGKGDPKKPR GKMSSYAFFV QTCREEHKKK HPDASVNFSE FSVR (SEQ ID NO: 354)
(355) MGKGDPKKPR GKMSSYAFFV QTCREEHKKK HPDASVNFSE FSVS (SEQ ID NO: 355)
(356) MGKGDPKKPR GKMSSYAFFV QTCREEHKKK HPDASVNFSE FSVT (SEQ ID NO: 356)
(357) MGKGDPKKPR GKMSSYAFFV QTCREEHKKK HPDASVNFSE FSVV (SEQ ID NO: 357)
(358) MGKGDPKKPR GKMSSYAFFV QTCREEHKKK HPDASVNFSE FSVW (SEQ ID NO: 358)
(359) MGKGDPKKPR GKMSSYAFFV QTCREEHKKK HPDASVNFSE FSVY (SEQ ID NO: 359)
(360) MGKGDPKKPR GKMSSYAFFV QTCREEHKKK HPDASVNFSE FSWA (SEQ ID NO: 360)
(361) MGKGDPKKPR GKMSSYAFFV QTCREEHKKK HPDASVNFSE FSWC (SEQ ID NO: 361)
(362) MGKGDPKKPR GKMSSYAFFV QTCREEHKKK HPDASVNFSE FSWD (SEQ ID NO: 362)
(363) MGKGDPKKPR GKMSSYAFFV QTCREEHKKK HPDASVNFSE FSWE (SEQ ID NO: 363)
(364) MGKGDPKKPR GKMSSYAFFV QTCREEHKKK HPDASVNFSE FSWF (SEQ ID NO: 364)
(365) MGKGDPKKPR GKMSSYAFFV QTCREEHKKK HPDASVNFSE FSWG (SEQ ID NO: 365)
(366) MGKGDPKKPR GKMSSYAFFV QTCREEHKKK HPDASVNFSE FSWH (SEQ ID NO: 366)
(367) MGKGDPKKPR GKMSSYAFFV QTCREEHKKK HPDASVNFSE FSWI (SEQ ID NO: 367)
(368) MGKGDPKKPR GKMSSYAFFV QTCREEHKKK HPDASVNFSE FSWK (SEQ ID NO: 368)
(369) MGKGDPKKPR GKMSSYAFFV QTCREEHKKK HPDASVNFSE FSWL (SEQ ID NO: 369)
(370) MGKGDPKKPR GKMSSYAFFV QTCREEHKKK HPDASVNFSE FSWM (SEQ ID NO: 370)
(371) MGKGDPKKPR GKMSSYAFFV QTCREEHKKK HPDASVNFSE FSWN (SEQ ID NO: 371)
(372) MGKGDPKKPR GKMSSYAFFV QTCREEHKKK HPDASVNFSE FSWP (SEQ ID NO: 372)
(373) MGKGDPKKPR GKMSSYAFFV QTCREEHKKK HPDASVNFSE FSWQ (SEQ ID NO: 373)
(374) MGKGDPKKPR GKMSSYAFFV QTCREEHKKK HPDASVNFSE FSWR (SEQ ID NO: 374)
(375) MGKGDPKKPR GKMSSYAFFV QTCREEHKKK HPDASVNFSE FSWS (SEQ ID NO: 375)
(376) MGKGDPKKPR GKMSSYAFFV QTCREEHKKK HPDASVNFSE FSWT (SEQ ID NO: 376)
(377) MGKGDPKKPR GKMSSYAFFV QTCREEHKKK HPDASVNFSE FSWV (SEQ ID NO: 377)
(378) MGKGDPKKPR GKMSSYAFFV QTCREEHKKK HPDASVNFSE FSWW (SEQ ID NO: 378)
(379) MGKGDPKKPR GKMSSYAFFV QTCREEHKKK HPDASVNFSE FSWY (SEQ ID NO: 379)
(380) MGKGDPKKPR GKMSSYAFFV QTCREEHKKK HPDASVNFSE FSYA (SEQ ID NO: 380)
(381) MGKGDPKKPR GKMSSYAFFV QTCREEHKKK HPDASVNFSE FSYC (SEQ ID NO: 381)
(382) MGKGDPKKPR GKMSSYAFFV QTCREEHKKK HPDASVNFSE FSYD (SEQ ID NO: 382)
(383) MGKGDPKKPR GKMSSYAFFV QTCREEHKKK HPDASVNFSE FSYE (SEQ ID NO: 383)
(384) MGKGDPKKPR GKMSSYAFFV QTCREEHKKK HPDASVNFSE FSYF (SEQ ID NO: 384)
(385) MGKGDPKKPR GKMSSYAFFV QTCREEHKKK HPDASVNFSE FSYG (SEQ ID NO: 385)
(386) MGKGDPKKPR GKMSSYAFFV QTCREEHKKK HPDASVNFSE FSYH (SEQ ID NO: 386)
(387) MGKGDPKKPR GKMSSYAFFV QTCREEHKKK HPDASVNFSE FSYI (SEQ ID NO: 387)
(388) MGKGDPKKPR GKMSSYAFFV QTCREEHKKK HPDASVNFSE FSYK (SEQ ID NO: 388)
(389) MGKGDPKKPR GKMSSYAFFV QTCREEHKKK HPDASVNFSE FSYL (SEQ ID NO: 389)
(390) MGKGDPKKPR GKMSSYAFFV QTCREEHKKK HPDASVNFSE FSYM (SEQ ID NO: 390)
(391) MGKGDPKKPR GKMSSYAFFV QTCREEHKKK HPDASVNFSE FSYN (SEQ ID NO: 391)
(392) MGKGDPKKPR GKMSSYAFFV QTCREEHKKK HPDASVNFSE FSYP (SEQ ID NO: 392)
(393) MGKGDPKKPR GKMSSYAFFV QTCREEHKKK HPDASVNFSE FSYQ (SEQ ID NO: 393)
(394) MGKGDPKKPR GKMSSYAFFV QTCREEHKKK HPDASVNFSE FSYR (SEQ ID NO: 394)
(395) MGKGDPKKPR GKMSSYAFFV QTCREEHKKK HPDASVNFSE FSYS (SEQ ID NO: 395)
(396) MGKGDPKKPR GKMSSYAFFV QTCREEHKKK HPDASVNFSE FSYT (SEQ ID NO: 396)
(397) MGKGDPKKPR GKMSSYAFFV QTCREEHKKK HPDASVNFSE FSYV (SEQ ID NO: 397)
(398) MGKGDPKKPR GKMSSYAFFV QTCREEHKKK HPDASVNFSE FSYW (SEQ ID NO: 398)
(399) MGKGDPKKPR GKMSSYAFFV QTCREEHKKK HPDASVNFSE FSYY (SEQ ID NO: 399)
(400) GKGDPKKPR GKMSSYAFFV QTCREEHKKK HPDASVNFSE FSPP (SEQ ID NO: 403)

The peptide of the present invention further includes a peptide having substitution of an amino acid at position 43 or/and position 44 of the peptide shown in the above SEQ ID NOs: 1 to 399 with a non-naturally occurring amino acid, and a peptide having substitution of an amino acid at position 42 or/and position 43 of the peptide shown in the above SEQ ID NO: 403 with a non-naturally occurring amino acid.

The peptide of the present invention may have a conservative substitution of an amino acid or amino acids in the peptide having the amino acid sequence shown in any one of SEQ ID NOs: 1 to 5. The conservative substitution refers to substitution with another amino acid having a structure or properties chemically similar to those of the original amino acid, without undergoing substantial changes in the functions of the peptide. Examples of the conservative substitution include mutual substitutions between aspartic acid and glutamic acid which are acidic amino acids; mutual substitutions between lysine, arginine, and histidine which are basic amino acids; mutual substitutions between serine, threonine, asparagine, and glutamine which are hydrophilic amino acids; and the like. For example, a peptide of SEQ ID NO: 167 is a product in which glutamic acid (E) at position 44 of the peptide of SEQ ID NO: 5 is substituted with aspartic acid (D), which is considered to have a function equivalent to that of the peptide of SEQ ID NO: 5. Further, peptides shown in SEQ ID NOs: 126, 128, 129, 138, 248, 254, 280, 282, 283 and 292 also have a conservative substitution of the amino acid in the peptide having the amino acid sequence shown in any one of SEQ ID NOs: 1 to 5, which are considered to have the function equivalent to that of any one of the peptide of SEQ ID NOs: 1 to 5. In one embodiment, the peptide of the present invention has the amino acid sequence shown in any one of SEQ ID NOs: 1 to 5, 126, 128, 129, 138, 167, 248, 254, 280, 282, 283, 292 and 403. In one more preferred embodiment, the peptide of the present invention has the amino acid sequence shown in any one of SEQ ID NOs: 1 to 5, 167 and 403, and in one even more preferred embodiment, the peptide of the present invention has the amino acid sequence shown in any one of SEQ ID NOs: 1 to 5 and 403, from the viewpoint that the peptide is expected to have the above function more surely.

Therefore, one of preferred embodiments of the peptide defined in the above (a) is a peptide having the amino acid sequence shown in any one of SEQ ID NOs: 1 to 5, 126, 128, 129, 138, 167, 248, 254, 280, 282, 283, 292 and 403, one of more preferred embodiment is a peptide having the amino acid sequence shown in any one of SEQ ID NOs: 1 to 5, 167 and 403, and one of even more preferred embodiment is a peptide having the amino acid sequence shown in any one of SEQ ID NOs: 1 to 5 and 403.

Further, one of preferred embodiments of the peptide defined in the above (b) is a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 5, 126, 128, 129, 138, 167, 248, 254, 280, 282, 283, 292 and 403, and having an activity of stimulating migration of cells, one of more preferred embodiment is a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 5, 167 and 403, and having an activity of stimulating migration of cells, and one of even more preferred embodiment is a peptide consisting of an amino acid sequence having addition of one or more amino acids, e.g., 2 to 40 amino acids, 2 to 30 amino acids, 2 to 20 amino acids, 2 to 10 amino acids, 2 to 5 amino acids, or 4, 3, or 2 amino acids, to the peptide having the amino acid sequence shown in any one of SEQ ID NOs: 1 to 5 and 403, and having an activity of stimulating migration of cells.

Further, one of preferred embodiments of the peptide as defined in the above (c) is a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 5, 126, 128, 129, 138, 167, 248, 254, 280, 282, 283 and 292 or an amino acid of position 1 to position 41 of the peptide having the amino acid sequence shown in SEQ ID NO: 403, and having an activity of stimulating migration of cells, one of more preferred embodiments is a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 5 and 167 or an amino acid of position 1 to position 41 of the peptide having the amino acid sequence shown in SEQ ID NO: 403, and having an activity of stimulating migration of cells, and one of even more preferred embodiments is a peptide comprising or consisting of an amino acid sequence having substitution, deletion, or insertion of one or more amino acids, e.g., 2 to 40 amino acids, 2 to 30 amino acids, 2 to 20 amino acids, 2 to 10 amino acids, 2 to 5 amino acids, or 4, 3, or 2 amino acids, at an amino acid of position 1 to position 42 of the peptide having the amino acid sequence shown in any one of SEQ ID NOs: 1 to 5 or an amino acid of position 1 to position 41 of the peptide having the amino acid sequence shown in SEQ ID NO: 403, and having an activity of stimulating migration of cells.

The term "derivative" of the peptide of the present invention means a peptide having substantially the same biological function or activity as the peptide of the present invention, in other words, the activity of stimulating migration of cells. The C-terminal of the derivative of the peptide may be any one of a carboxyl group, a carboxylate, an amide, or an ester. For example, the derivative includes a modified peptide obtained by addition of a modifying group, a mutant peptide obtained by altering an amino acid residue, and the like.

The modifying group includes functional groups showing fluorescent properties, functional groups not involved in the formation of conformation of a peptide, and the like. In addition, the modifying group may be an amino acid residue (for example, β-alanine etc.) other than an amino acid residue constituting the naturally occurring peptide of the present invention. The functional group showing fluorescent properties includes eosin, fluorescein isothiocyanate (FITC) and the like. The functional groups not involved in the formation of conformation of a peptide include spacer groups represented by β-alanine residues, and the like. It is preferable that the functional group is present at a terminal. Further, a peptide modified with polyethylene glycol (PEG) is also included in the "derivative."

The term "pharmaceutically acceptable salt" of the peptide of the present invention includes physiologically acceptable salts with acids or bases, and particularly acid-added salts are preferred. As the salt as mentioned above, a salt with an inorganic acid such as hydrochloric acid, phosphoric acid, hydrobromic acid, or sulfuric acid, a salt with an organic acid such as acetic acid, formic acid, propionic acid, fumaric acid, maleic acid, succinic acid, tartaric acid, citric acid, malic acid, oxalic acid, benzoic acid, methanesulfonic acid, or benzenesulfonic acid, or the like is used.

The term "solvate" of the peptide of the present invention or the above salt includes those in which any number of solvent molecules are coordinated to the peptide of the present invention, and preferably a hydrate.

The present invention provides the peptide as described above. In the synthesis of the peptide in the present invention, the peptide can be chemically synthesized according to any of the methods of the peptide liquid phase synthesis methods and the peptide solid phase synthesis methods. In the present invention, the peptide synthesized according to the peptide solid phase synthesis method is preferred. The peptide solid phase synthesis method is one of the generally employed methods when peptides are chemically synthesized, which can be, for example, carried out as follows: Using as a solid phase beads of polystyrene polymer gel having a diameter of 0.1 mm or so of which surfaces are modified with amino groups, an amino acid chain is extended one by one by dehydration reaction therefrom. When an intended sequence of the peptide is prepared, the peptide is cut out from the solid phase surface, to obtain an intended substance.

According to the solid phase synthesis method, it is made possible to perform the synthesis of ribosomal peptide which is difficult to synthesize in bacteria, introduction of a non-naturally occurring amino acid such as D-form or heavy atom substituted compound, modification of peptide and protein main chain, or the like. When a long peptide chain of from 70 to exceeding 100 peptides is synthesized in the solid phase method, the peptide chains can be synthesized by binding two peptide chains using a native chemical ligation method.

The method for administration of a composition of the present invention includes oral administration or parenteral administration, and specific methods of the parenteral administration include injection administration, transnasal administration, transpulmonary administration, transdermal administration, and the like, but not limited thereto. As examples of the injection administration, for example, a composition of the present invention can be administered systemically or locally (e.g., subcutaneously, intradermally, epidermally, to eyeball or palpebral conjunctiva, to nasal cavity mucosa, to oral cavity and gastrointestinal mucosa, to vaginal or intrauterine mucosa, to a damaged site, etc.) by intravenous injection, intramuscular injection, intraperitoneal injection, subcutaneous injection or the like.

In addition, the method for administration of a composition of the present invention includes, for example, intravascular administration (intraarterial administration, intravenous administration, etc.), blood administration, intramuscular administration, subcutaneous administration, intradermal administration, and intraperitoneal administration, but not limited thereto.

In addition, the site of administration is not limited, which includes a tissue site in need of regeneration or its neighborhood region, a site different from the tissue in need of regeneration, or a site distal and different from the tissue in need of regeneration. For example, the site of administration includes blood (intraarterially, intravenously, etc.), muscles, subcutaneous site, intradermal site and intraperitoneal site, but not limited thereto.

In addition, the method for administration can be appropriately selected depending upon age and symptoms of the patients. When the peptide of the present invention is administered, for example, a dose within a range of from 0.0000001 mg to 1,000 mg per 1 kg body weight per single administration can be selected. Alternatively, for example, a dose within a range of from 0.00001 to 100,000 mg/body per patient can be selected. When cells that secrete the peptide of the present invention or a vector for genetic therapy into which a DNA encoding the peptide is inserted is administered, the peptide can be administered such that the amount of the peptide is within the above range. However, the pharmaceutical composition of the present invention is not limited to these doses.

The pharmaceutical composition of the present invention can be formulated in accordance with conventional methods (e.g., Remington's Pharmaceutical Science, latest edition, Mark Publishing Company, Easton, U.S.A), which may together contain a pharmaceutically acceptable carrier or additives. Examples of the carriers and additives include, for example, surfactants, excipients, colorants, perfumes, preservatives, stabilizers, buffers, suspending agents, isotonic agents, binders, disintegrants, lubricants, fluidity promoters, flavoring agents, and the like, but not limited thereto, and other conventionally used carriers can be appropriately used. Specific examples include light anhydrous silicic acid, lactose, crystalline celluloses, mannitol, starches, carmellose calcium, carmellose sodium, hydroxypropyl cellulose, hydroxypropylmethyl cellulose, polyvinyl acetal diethylaminoacetate, polyvinyl pyrrolidone, gelatin, medium-chained fatty acid triglyceride, polyoxyethylene hydrogenated castor oil 60, white sugar, carboxymethyl cellulose, corn starch, inorganic salts, and the like.

Here, all the prior art references cited in the present specification will be incorporated herein by reference.

### EXAMPLES

The present invention will be further illustrated by means of the following Examples, without intending to limit the present invention to the following Examples.

### Example 1

### Synthesis of Various Kinds of HMGB1 Peptides

A peptide consisting of the amino acids at position 2 to position 44 of human HMGB1 (2-44) and each of the five kinds of peptides having substitution of an amino acid sequence at position 43 and/or position 44 in the peptide at position 1 to position 44 of human HMGB1 (Peptide-1 to -5) were synthesized by a solid phase method.

2-44 GKGDPKKPR GKMSSYAFFV QTCREEHKKK
HPDASVNFSE FSKK (SEQ ID NO: 400)

Peptide-1 MGKGDPKKPR GKMSSYAFFV QTCREEHKKK
HPDASVNFSE FSKP (SEQ ID NO: 1)

Peptide-2 MGKGDPKKPR GKMSSYAFFV QTCREEHKKK
HPDASVNFSE FSPK (SEQ ID NO: 2)

Peptide-3 MGKGDPKKPR GKMSSYAFFV QTCREEHKKK
HPDASVNFSE FSPP (SEQ ID NO: 3)

Peptide-4 MGKGDPKKPR GKMSSYAFFV QTCREEHKKK
HPDASVNFSE FSKA (SEQ ID NO: 4)

Peptide-5 MGKGDPKKPR GKMSSYAFFV QTCREEHKKK
HPDASVNFSE FSKE (SEQ ID NO: 5)

### Example 2

### Measurements of Cell-Migration Activity

Cells of murine bone marrow-derived stromal cell line ST2 (RCB0224, RIKEN BioResource Research Center) were removed from a dish using TrypLE Express (Thermo Fisher Scientific), and collected by centrifugation at 25°C and 1,500 rpm for 5 minutes. The cell pellets were prepared into a concentration of 7 x 10⁵ cells/mL with RPMI 1640 / 10% FBS / 1% PenStrep (Gibco), and seeded at 70 µL/insert well on a 24-well plate set with a culture insert (Nippon Genetics Co., Ltd.). After 24 hours, the culture insert was taken out, and the cells were washed once with RPMI 1640. Each of the peptide samples (a peptide of position 2 to position 44 of human HMGB1 (2-44), and the five kinds of modified peptides Peptide-1 to -5 prepared in Example 1), each prepared at 10 µM with an assay medium (RPMI 1640 / 0.1% FBS) was added in an amount of 550 µL, and the cells were further cultured overnight. As a negative control (NC), only the assay medium was added. After the culture, the cells were fixed, stained with DiffQuik, and photographed. The degree of cell migration of each group from the photographed images was evaluated with five levels of scores (-, ±, +, ++, +++). In addition, each of the peptide samples was subjected to three test runs under the same conditions.

### Results

The presence or absence of the migration activity of the peptide of position 2 to position 44 of human HMGB1 (2-44), the five kinds of modified peptides Peptide-1 to -5 prepared in Example 1, and NC was ascertained. As a result, all of the five kinds of modified peptides were found to have migration activity (Table 1).

### [Table 1]

**Table 1**

| | 1 st Run | 2nd Run | 3rd Run |
|---|---|---|---|
| NC | - | ± | - |
| 2-44 | +++ | +++ | +++ |
| Peptide-1 | +++ | + | +++ |
| Peptide-2 | +++ | ++ | +++ |
| Peptide-3 | + | + | + |
| Peptide-4 | ++ | + | +++ |
| Peptide-5 | ++ | ++ | ++ |

### Example 3

### Stability of Peptides in Plasma

Whole blood collected from Crl : CD (SD) rats using an anticoagulant EDTA-2K was centrifuged to obtain a blank plasma. The plasma obtained was stored on ice, and used only on the day of the stability test.

Each of the six kinds of peptides used in Example 2 was prepared into a solution at 375 µg/mL with distilled water from a stock solution in which each peptide was dissolved in a 0.1% aqueous TFA solution at 1 mg/mL. Sixteen microliters of the solution was added to 1,184 µL of the blank plasma to prepare a sample for the stability test (assay concentration: 5 µg/mL). The sample for stability test was allowed to stand on ice, and 50 µL (at each time point, 3 samples) was collected immediately after the addition, and 1, 3, 5, 15, 30, and 60 minutes after the addition. To 50 µL of the collected sample were added 250 µL of 270 mmol/L citric acid solution / 130 mmol/L EDTA-2K aqueous solution (4:1, v/v), 25 µL of a 0.1% aqueous TFA solution, and 25 µL of IS solution (4 µg/mL aqueous 0.1% TFA solution of a compound in which nine carbon atoms and one nitrogen atom of phenylalanine at position 18, position 38, and position 41 in the peptide of position 1 to position 44 of human HMGB1 are each substituted with a stable isotope), and mixed. To the mixture was added 350 µL of methanol and mixed, and further 100 µL of acetonitrile was added thereto and mixed, and the mixture was once frozen with dry ice. The frozen mixture was thawed at room temperature, and the supernatant obtained by centrifugation was transferred to a separate container and stirred to be used as a sample for the measurement.

The samples for the measurement were introduced into high-performance liquid chromatograph-tandem quadrupolar mass spectrometer to detect the monitor ions peculiar to each of the peptides. From separately prepared samples of which concentrations were known, a calibration curve was drawn in a concentration range of from 0.1 to 10 µg/mL, and an inverse regression was obtained from peak areas of chromatograms obtained by measurements of each sample to obtain a peptide concentration in the samples. The peptide concentration at each time point was averaged, and a remaining rate (%) which was calculated by defining one immediately after the addition as 100 was plotted against the time after the addition (FIG. 1).

### Results

As a result of the stability test in rat plasma (on ice) of the peptide at position 2 to position 44 of human HMGB1 (2-44) and the five kinds of peptides in which the amino acids at position 43 and position 44 were modified (Peptide-1 to -5), 2-44 were equal to or lower than the quantifiable lower limit (0.5 µg/mL) for the sample at 15 minutes or more later of the addition. On the other hand, it was confirmed that the five kinds of the modified peptides Peptide-1 to -5 all were stable as compared to 2-44 (FIG. 1).

### Example 4

### Synthesis of HMGB1 Peptide

A peptide consisting of a sequence given below having substitution of two amino acids at a C-terminal in the peptide of position 2 to position 44 of human HMGB1 (2-44) (Peptide-6) was synthesized by a solid phase method.

Peptide-6 GKGDPKKPR GKMSSYAFFV QTCREEHKKK
HPDASVNFSE FSPP (SEQ ID NO: 403)

### Example 5

### Measurements of Cell-Migration Activity

Cells of murine bone marrow-derived stromal cell line ST2 (RCB0224, RIKEN BioResource Research Center) were removed from a dish using 2.5g/L - Trypsin/1mmol/L - EDTA Solution (nacalai tesque), and collected by centrifugation at 25°C and 200 g for 3 minutes. The cell pellets were prepared into a concentration of 8.6 x 10⁵ cells/mL with RPMI 1640 / 10% FBS / 1% PenStrep (nacalai tesque), and seeded at 70 µL/insert well on a 24-well plate set with a culture insert (Nippon Genetics Co., Ltd.). After 18 hours, the culture insert was taken out, and the cells were washed once with RPMI 1640. Each of the peptide samples (the peptide of position 2 to position 44 of human HMGB1 (2-44), and the modified peptide Peptide-6 prepared in Example 4), each prepared at 20 µM with an assay medium (RPMI 1640 / 0.1% FBS) was added in an amount of 1,000 µL, and the cells were cultured for additional 20 hours. As a negative control (NC), only the assay medium was added. Immediately after the addition of the assay medium and after 20 hours of culture, the cells were photographed. From the photographed image, the degree of cell migration of each group was evaluated as a proportion (%) of the area occupied by the cells migrated to the region at 20 hours relative to the area of blank region prepared by the culture insert (region in which the cells do not exist at the time point of addition of the assay medium).

### Results

The presence or absence of the migration activity of the peptide at position 2 to position 44 of human HMGB1 (2-44), the modified peptide prepared in Example 4 (Peptide-6) and NC was ascertained. As a result, the modified peptide (Peptide-6) was found to have the same level of migration activity as the peptide at position 2 to position 44 of human HMGB1 (2-44) (FIG. 2).

### Example 6

### Stability of Peptides in Plasma

A 5 mL tube previously charged with 60 µL of a 10% EDTA-2K was charged with 3 mL of whole blood collected from Crl: CD (SD) rats, and the contents were allowed to stand at room temperature for 30 minutes, and then centrifuged at 4°C and at 3,000 rpm (1600 × g) for 10 minutes. The supernatant was collected to be used as a blank plasma. The plasma obtained was stored on ice and only used on the day of the stability test.

Each of the peptide at position 2 to position 44 of human HMGB1 (2-44), the modified peptide prepared in Example 1 (Peptide-3), and the modified peptide prepared in Example 4 (Peptide-6) was prepared into a solution at 375 µg/mL with distilled water from a stock solution in which each peptide was dissolved in a 0.1% aqueous TFA solution at 1 mg/mL. Eight microliters of the solution was added to 592 µL of the blank plasma to prepare a sample for the stability test (assay concentration: 5 µg/mL). The sample for stability test was allowed to stand on ice, and 50 µL was collected immediately after the addition, and 30 and 60 minutes after the addition. To 50 µL of the collected sample were added 250 µL of 270 mmol/L citric acid solution / 130 mmol/L EDTA-2K aqueous solution (4:1, v/v), and 50 µL of a 0.1% aqueous TFA solution, and mixed. To the mixture was added 350 µL of methanol and mixed, and further 100 µL of acetonitrile was added thereto and mixed, and the mixture was once frozen with dry ice. After 5 to 60 minutes, the frozen sample was thawed at room temperature, and the supernatant obtained by centrifugation at 4°C and at 14,000 × g for 3 minutes was collected in an amount of 400 µL, dried and solidified with a centrifugal concentrator (Spin Dryer Light VC-36R, manufactured by TAITEC CORPORATION), and dissolved in 200 µL of a 1% TFA / 2% ACN Buffer, and filtrated with a filter (0.22 µm) to obtain samples for the measurement.

The samples for the measurement were introduced into high-performance liquid chromatograph-single quadrupolar mass spectrometer to detect the monitor ions peculiar to each of the peptides. From separately prepared samples of which concentrations were known, a calibration curve was drawn in a concentration range of from 0.1 to 10 µg/mL, and an inverse regression was obtained from peak areas of chromatograms obtained by measurements of each sample to obtain a peptide concentration in the samples. For the peptide concentration at each time point, a remaining rate (%) which was calculated by defining one immediately after the addition as 100 was plotted against the time after the addition (FIG. 3).

### Results

As a result of the stability test in rat plasma (on ice), 2-44 showed to be equal to or lower than the quantifiable lower limit (0.5 µg/mL) at a time point of 30 minutes after the addition. On the other hand, it was confirmed that both of Peptide-3 and Peptide-6 were stable as compared to 2-44. In addition, the stability of Peptide-3 and Peptide-6 was found to be of the same level (FIG. 3).

### INDUSTRIAL APPLICABILITY

The peptide, a derivative thereof, a pharmaceutically acceptable salt thereof, or a solvate thereof of the present invention is used in the field of regenerative medicine.

## Claims

1. A peptide selected from the group consisting of:
a peptide having the amino acid sequence shown in any one of SEQ ID NOs: 1 to 399 and 403,
a peptide consisting of an amino acid sequence having addition of one or more amino acids to the peptide having the amino acid sequence shown in any one of SEQ ID NOs: 1 to 399 and 403, and having an activity of stimulating migration of cells, and
a peptide comprising or consisting of an amino acid sequence having substitution, deletion, or insertion of one or more amino acids at an amino acid of position 1 to position 42 of the peptide having the amino acid sequence shown in any one of SEQ ID NOs: 1 to 399 or at an amino acid of position 1 to position 41 of the peptide having the amino acid sequence shown in SEQ ID NO: 403, and having an activity of stimulating migration of cells,
a derivative thereof, a pharmaceutically acceptable salt thereof, or a solvate thereof.

2. A peptide selected from the group consisting of:
a peptide having the amino acid sequence shown in any one of SEQ ID NOs: 1 to 5, 126, 128, 129, 138, 167, 248, 254, 280, 282, 283, 292 and 403,
a peptide consisting of an amino acid sequence having addition of one or more amino acids to the peptide having the amino acid sequence shown in anyone of SEQ ID NOs: 1 to 5, 126, 128, 129, 138, 167, 248, 254, 280, 282, 283, 292 and 403, and having an activity of stimulating migration of cells, and
a peptide comprising or consisting of an amino acid sequence having substitution, deletion, or insertion of one or more amino acids at an amino acid of position 1 to position 42 of the peptide having the amino acid sequence shown in any one of SEQ ID NOs: 1 to 5, 126, 128, 129, 138, 167, 248, 254, 280, 282, 283 and 292, or at an amino acid of position 1 to position 41 of the peptide having the amino acid sequence shown in SEQ ID NO: 403, and having an activity of stimulating migration of cells, a derivative thereof, a pharmaceutically acceptable salt thereof, or a solvate thereof.

3. A peptide selected from the group consisting of:
a peptide having the amino acid sequence shown in any one of SEQ ID NOs: 1 to 5, 167 and 403,
a peptide consisting of an amino acid sequence having addition of one or more amino acids to the peptide having the amino acid sequence shown in any one of SEQ ID NOs: 1 to 5, 167 and 403, and having an activity of stimulating migration of cells, and
a peptide comprising or consisting of an amino acid sequence having substitution, deletion, or insertion of one or more amino acids at an amino acid of position 1 to position 42 of the peptide having the amino acid sequence shown in any one of SEQ ID NOs: 1 to 5 and 167 or at an amino acid of position 1 to position 41 of the peptide having the amino acid sequence shown in SEQ ID NO: 403, and having an activity of stimulating migration of cells,
a derivative thereof, a pharmaceutically acceptable salt thereof, or a solvate thereof.

4. A peptide selected from the group consisting of:
a peptide having the amino acid sequence shown in any one of SEQ ID NOs: 1 to 5 and 403,
a peptide consisting of an amino acid sequence having addition of one or more amino acids to the peptide having the amino acid sequence shown in any one of SEQ ID NOs: 1 to 5 and 403, and having an activity of stimulating migration of cells, and
a peptide comprising or consisting of an amino acid sequence having substitution, deletion, or insertion of one or more amino acids at an amino acid of position 1 to position 42 of the peptide having the amino acid sequence shown in any one of SEQ ID NOs: 1 to 5 or at an amino acid of position 1 to position 41 of the peptide having the amino acid sequence shown in SEQ ID NO: 403, and having an activity of stimulating migration of cells,
a derivative thereof, a pharmaceutically acceptable salt thereof, or a solvate thereof.

5. A composition for use in stimulation of migration of PDGFRα-positive cells, comprising a peptide, a derivative thereof, a pharmaceutically acceptable salt thereof, or a solvate thereof as defined in any one of the claims 1 to 4.

6. A composition for use in recruitment of bone marrow mesenchymal stem cells from bone marrow to peripheral blood, comprising a peptide, a derivative thereof, a pharmaceutically acceptable salt thereof, or a solvate thereof as defined in any one of the claims 1 to 4.

7. A composition for use in regeneration of tissues, comprising a peptide, a derivative thereof, a pharmaceutically acceptable salt thereof, or a solvate thereof as defined in any one of the claims 1 to 4.

8. A composition for use in stimulation of migration of bone marrow-derived stromal cells, comprising a peptide, a derivative thereof, a pharmaceutically acceptable salt thereof, or a solvate thereof as defined in any one of the claims 1 to 4.


SEQUENCE LISTING

<110>  SHIONOGI & CO.,LTD
       GENOMIX CO.,LTD

<120>  Peptide for inducing regeneration of tissue and use thereof

<130>  18-010-PCTJP

<150>  JP 2017-085793
<151>  2017-04-25

<160>  403

<170>  PatentIn version 3.5

<210>  1
<211>  44
<212>  PRT
<213>  Artificial Sequence

<220>
<223>

<400>  1

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Pro
        35                  40

<210>  2
<211>  44
<212>  PRT
<213>  Artificial Sequence

<220>
<223>

<400>  2

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Lys
        35                  40

<210>  3
<211>  44
<212>  PRT

<213>  Artificial Sequence

<220>
<223>

<400>  3

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Pro
        35                  40

<210>  4
<211>  44
<212>  PRT
<213>  Artificial Sequence

<220>
<223>

<400>  4

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Ala
        35                  40

<210>  5
<211>  44
<212>  PRT
<213>  Artificial Sequence

<220>
<223>

<400>  5

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Glu
        35                  40

<210>  6
<211>  44
<212>  PRT
<213>  Artificial Sequence

<220>
<223>

<400>  6

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Ala
        35                  40

<210>  7
<211>  44
<212>  PRT
<213>  Artificial Sequence

<220>
<223>

<400>  7

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Cys
        35                  40

<210>  8
<211>  44
<212>  PRT
<213>  Artificial Sequence

<220>
<223>

<400>  8

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Asp

35                  40

<210>  9
<211>  44
<212>  PRT
<213>  Artificial Sequence

<220>
<223>

<400>  9

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Glu
        35                  40

<210>  10
<211>  44
<212>  PRT
<213>  Artificial Sequence

<220>
<223>

<400>  10

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Phe
        35                  40

<210>  11
<211>  44
<212>  PRT
<213>  Artificial Sequence

<220>
<223>

<400>  11

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Gly
        35                  40

<210>  12
<211>  44
<212>  PRT
<213>  Artificial Sequence

<220>
<223>

<400>  12

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala His
        35                  40

<210>  13
<211>  44
<212>  PRT
<213>  Artificial Sequence

<220>
<223>

<400>  13

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Ile
        35                  40

<210>  14
<211>  44
<212>  PRT
<213>  Artificial Sequence

<220>
<223>

<400>  14

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
                20                  25                  30

Asp Ala Ser Val Asn Phe Ser Glu Phe Ser Ala Lys
            35                  40

<210>  15
<211>  44
<212>  PRT
<213>  Artificial Sequence

<220>
<223>

<400>  15

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
                20                  25                  30

Asp Ala Ser Val Asn Phe Ser Glu Phe Ser Ala Leu
            35                  40

<210>  16
<211>  44
<212>  PRT
<213>  Artificial Sequence

<220>
<223>

<400>  16

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
                20                  25                  30

Asp Ala Ser Val Asn Phe Ser Glu Phe Ser Ala Met
            35                  40

<210>  17
<211>  44
<212>  PRT
<213>  Artificial Sequence

<220>
<223>

<400>  17

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Asn
        35                  40

<210>  18
<211>  44
<212>  PRT
<213>  Artificial Sequence

<220>
<223>

<400>  18

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Pro
        35                  40

<210>  19
<211>  44
<212>  PRT
<213>  Artificial Sequence

<220>
<223>

<400>  19

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Gln
        35                  40

<210>  20
<211>  44
<212>  PRT
<213>  Artificial Sequence

<220>
<223>

<400>  20

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Arg
        35                  40

<210>  21
<211>  44
<212>  PRT
<213>  Artificial Sequence

<220>
<223>

<400>  21

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Ser
        35                  40

<210>  22
<211>  44
<212>  PRT
<213>  Artificial Sequence

<220>
<223>

<400>  22

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Thr
        35                  40

<210>  23
<211>  44
<212>  PRT
<213>  Artificial Sequence

<220>
<223>

<400>  23

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Val
        35                  40

<210>  24
<211>  44
<212>  PRT
<213>  Artificial Sequence

<220>
<223>

<400>  24

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Trp
        35                  40

<210>  25
<211>  44
<212>  PRT
<213>  Artificial Sequence

<220>
<223>

<400>  25

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ala Tyr
        35                  40

<210>  26

<211>  44
<212>  PRT
<213>  Artificial Sequence

<220>
<223>

<400>  26

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Ala
        35                  40

<210>  27
<211>  44
<212>  PRT
<213>  Artificial Sequence

<220>
<223>

<400>  27

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Cys
        35                  40

<210>  28
<211>  44
<212>  PRT
<213>  Artificial Sequence

<220>
<223>

<400>  28

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Asp
        35                  40

<210>  29
<211>  44
<212>  PRT
<213>  Artificial Sequence

<220>
<223>

<400>  29

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Glu
        35                  40

<210>  30
<211>  44
<212>  PRT
<213>  Artificial Sequence

<220>
<223>

<400>  30

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Phe
        35                  40

<210>  31
<211>  44
<212>  PRT
<213>  Artificial Sequence

<220>
<223>

<400>  31

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Gly
        35                  40

<210>  32
<211>  44
<212>  PRT
<213>  Artificial Sequence

<220>
<223>

<400>  32

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys His
        35                  40

<210>  33
<211>  44
<212>  PRT
<213>  Artificial Sequence

<220>
<223>

<400>  33

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Ile
        35                  40

<210>  34
<211>  44
<212>  PRT
<213>  Artificial Sequence

<220>
<223>

<400>  34

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Lys
        35                  40

<210>  35
<211>  44
<212>  PRT
<213>  Artificial Sequence

<220>
<223>

<400>  35

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Leu
        35                  40

<210>  36
<211>  44
<212>  PRT
<213>  Artificial Sequence

<220>
<223>

<400>  36

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Met
        35                  40

<210>  37
<211>  44
<212>  PRT
<213>  Artificial Sequence

<220>
<223>

<400>  37

Met Gly Lys Gly Asp Pro Lys Lys Pro Arg Gly Lys Met Ser Ser Tyr

1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Asn
        35                  40

<210>  38
<211>  44
<212>  PRT
<213>  Artificial Sequence

<220>
<223>

<400>  38

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Pro
        35                  40

<210>  39
<211>  44
<212>  PRT
<213>  Artificial Sequence

<220>
<223>

<400>  39

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Gln
        35                  40

<210>  40
<211>  44
<212>  PRT
<213>  Artificial Sequence

<220>
<223>

<400>  40

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Arg
        35                  40

<210>  41
<211>  44
<212>  PRT
<213>  Artificial Sequence

<220>
<223>

<400>  41

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Ser
        35                  40

<210>  42
<211>  44
<212>  PRT
<213>  Artificial Sequence

<220>
<223>

<400>  42

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Thr
        35                  40

<210>  43
<211>  44
<212>  PRT
<213>  Artificial Sequence

<220>
<223>

<400>  43

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Val
        35                  40

<210>  44
<211>  44
<212>  PRT
<213>  Artificial Sequence

<220>
<223>

<400>  44

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Trp
        35                  40

<210>  45
<211>  44
<212>  PRT
<213>  Artificial Sequence

<220>
<223>

<400>  45

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Cys Tyr
        35                  40

<210>  46
<211>  44

<212>  PRT
<213>  Artificial Sequence

<220>
<223>

<400>  46

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Ala
        35                  40

<210>  47
<211>  44
<212>  PRT
<213>  Artificial Sequence

<220>
<223>

<400>  47

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Cys
        35                  40

<210>  48
<211>  44
<212>  PRT
<213>  Artificial Sequence

<220>
<223>

<400>  48

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Asp
        35                  40

<210>  49
<211>  44
<212>  PRT
<213>  Artificial Sequence

<220>
<223>

<400>  49

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Glu
        35                  40

<210>  50
<211>  44
<212>  PRT
<213>  Artificial Sequence

<220>
<223>

<400>  50

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Phe
        35                  40

<210>  51
<211>  44
<212>  PRT
<213>  Artificial Sequence

<220>
<223>

<400>  51

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Gly
        35                  40

<210>  52
<211>  44
<212>  PRT
<213>  Artificial Sequence

<220>
<223>

<400>  52

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp His
        35                  40

<210>  53
<211>  44
<212>  PRT
<213>  Artificial Sequence

<220>
<223>

<400>  53

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Ile
        35                  40

<210>  54
<211>  44
<212>  PRT
<213>  Artificial Sequence

<220>
<223>

<400>  54

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro

20                  25                  30

Asp Ala Ser Val Asn Phe Ser Glu Phe Ser Asp Lys
        35                  40

<210>  55
<211>  44
<212>  PRT
<213>  Artificial Sequence

<220>
<223>

<400>  55

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Leu
        35                  40

<210>  56
<211>  44
<212>  PRT
<213>  Artificial Sequence

<220>
<223>

<400>  56

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Met
        35                  40

<210>  57
<211>  44
<212>  PRT
<213>  Artificial Sequence

<220>
<223>

<400>  57

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Asn
        35                  40

<210>  58
<211>  44
<212>  PRT
<213>  Artificial Sequence

<220>
<223>

<400>  58

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Pro
        35                  40

<210>  59
<211>  44
<212>  PRT
<213>  Artificial Sequence

<220>
<223>

<400>  59

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Gln
        35                  40

<210>  60
<211>  44
<212>  PRT
<213>  Artificial Sequence

<220>
<223>

<400>  60

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Arg
        35                  40

<210>  61
<211>  44
<212>  PRT
<213>  Artificial Sequence

<220>
<223>

<400>  61

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Ser
        35                  40

<210>  62
<211>  44
<212>  PRT
<213>  Artificial Sequence

<220>
<223>

<400>  62

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Thr
        35                  40

<210>  63
<211>  44
<212>  PRT
<213>  Artificial Sequence

<220>

<223>

<400>  63

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Val
        35                  40

<210>  64
<211>  44
<212>  PRT
<213>  Artificial Sequence

<220>
<223>

<400>  64

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Trp
        35                  40

<210>  65
<211>  44
<212>  PRT
<213>  Artificial Sequence

<220>
<223>

<400>  65

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asp Tyr
        35                  40

<210>  66
<211>  44
<212>  PRT

<213>  Artificial Sequence

<220>
<223>

<400>  66

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Ala
        35                  40

<210>  67
<211>  44
<212>  PRT
<213>  Artificial Sequence

<220>
<223>

<400>  67

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Cys
        35                  40

<210>  68
<211>  44
<212>  PRT
<213>  Artificial Sequence

<220>
<223>

<400>  68

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Asp
        35                  40

<210>  69
<211>  44
<212>  PRT
<213>  Artificial Sequence

<220>
<223>

<400>  69

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Glu
        35                  40

<210>  70
<211>  44
<212>  PRT
<213>  Artificial Sequence

<220>
<223>

<400>  70

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Phe
        35                  40

<210>  71
<211>  44
<212>  PRT
<213>  Artificial Sequence

<220>
<223>

<400>  71

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Gly

35                  40

<210>  72
<211>  44
<212>  PRT
<213>  Artificial Sequence

<220>
<223>

<400>  72

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu His
        35                  40

<210>  73
<211>  44
<212>  PRT
<213>  Artificial Sequence

<220>
<223>

<400>  73

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Ile
        35                  40

<210>  74
<211>  44
<212>  PRT
<213>  Artificial Sequence

<220>
<223>

<400>  74

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Lys
        35                  40

<210>  75
<211>  44
<212>  PRT
<213>  Artificial Sequence

<220>
<223>

<400>  75

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Leu
        35                  40

<210>  76
<211>  44
<212>  PRT
<213>  Artificial Sequence

<220>
<223>

<400>  76

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Met
        35                  40

<210>  77
<211>  44
<212>  PRT
<213>  Artificial Sequence

<220>
<223>

<400>  77

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Asn
        35                  40

<210>  78
<211>  44
<212>  PRT
<213>  Artificial Sequence

<220>
<223>

<400>  78

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Pro
        35                  40

<210>  79
<211>  44
<212>  PRT
<213>  Artificial Sequence

<220>
<223>

<400>  79

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Gln
        35                  40

<210>  80
<211>  44
<212>  PRT
<213>  Artificial Sequence

<220>
<223>

<400>  80

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Arg
        35                  40

<210>  81
<211>  44
<212>  PRT
<213>  Artificial Sequence

<220>
<223>

<400>  81

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Ser
        35                  40

<210>  82
<211>  44
<212>  PRT
<213>  Artificial Sequence

<220>
<223>

<400>  82

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Thr
        35                  40

<210>  83
<211>  44
<212>  PRT
<213>  Artificial Sequence

<220>
<223>

<400>  83

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Val
        35                  40

<210>  84
<211>  44
<212>  PRT
<213>  Artificial Sequence

<220>
<223>

<400>  84

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Trp
        35                  40

<210>  85
<211>  44
<212>  PRT
<213>  Artificial Sequence

<220>
<223>

<400>  85

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Glu Tyr
        35                  40

<210>  86
<211>  44
<212>  PRT
<213>  Artificial Sequence

<220>
<223>

<400>  86

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Ala
        35                  40

<210>  87
<211>  44
<212>  PRT
<213>  Artificial Sequence

<220>
<223>

<400>  87

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Cys
        35                  40

<210>  88
<211>  44
<212>  PRT
<213>  Artificial Sequence

<220>
<223>

<400>  88

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Asp
        35                  40

<210>  89

<211>  44
<212>  PRT
<213>  Artificial Sequence

<220>
<223>

<400>  89

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Glu
        35                  40

<210>  90
<211>  44
<212>  PRT
<213>  Artificial Sequence

<220>
<223>

<400>  90

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Phe
        35                  40

<210>  91
<211>  44
<212>  PRT
<213>  Artificial Sequence

<220>
<223>

<400>  91

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Gly
        35                  40

<210>  92
<211>  44
<212>  PRT
<213>  Artificial Sequence

<220>
<223>

<400>  92

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe His
        35                  40

<210>  93
<211>  44
<212>  PRT
<213>  Artificial Sequence

<220>
<223>

<400>  93

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Ile
        35                  40

<210>  94
<211>  44
<212>  PRT
<213>  Artificial Sequence

<220>
<223>

<400>  94

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Lys
        35                  40

<210>  95
<211>  44
<212>  PRT
<213>  Artificial Sequence

<220>
<223>

<400>  95

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Leu
        35                  40

<210>  96
<211>  44
<212>  PRT
<213>  Artificial Sequence

<220>
<223>

<400>  96

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Met
        35                  40

<210>  97
<211>  44
<212>  PRT
<213>  Artificial Sequence

<220>
<223>

<400>  97

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Asn
        35                  40

<210>  98
<211>  44
<212>  PRT
<213>  Artificial Sequence

<220>
<223>

<400>  98

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Pro
        35                  40

<210>  99
<211>  44
<212>  PRT
<213>  Artificial Sequence

<220>
<223>

<400>  99

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Gln
        35                  40

<210>  100
<211>  44
<212>  PRT
<213>  Artificial Sequence

<220>
<223>

<400>  100

Met Gly Lys Gly Asp Pro Lys Lys Pro Arg Gly Lys Met Ser Ser Tyr

1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Arg
        35                  40

<210>  101
<211>  44
<212>  PRT
<213>  Artificial Sequence

<220>
<223>

<400>  101

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Ser
        35                  40

<210>  102
<211>  44
<212>  PRT
<213>  Artificial Sequence

<220>
<223>

<400>  102

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Thr
        35                  40

<210>  103
<211>  44
<212>  PRT
<213>  Artificial Sequence

<220>
<223>

<400>  103

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Val
        35                  40

<210>  104
<211>  44
<212>  PRT
<213>  Artificial Sequence

<220>
<223>

<400>  104

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Trp
        35                  40

<210>  105
<211>  44
<212>  PRT
<213>  Artificial Sequence

<220>
<223>

<400>  105

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Phe Tyr
        35                  40

<210>  106
<211>  44
<212>  PRT
<213>  Artificial Sequence

<220>
<223>

<400>  106

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Ala
        35                  40

<210>  107
<211>  44
<212>  PRT
<213>  Artificial Sequence

<220>
<223>

<400>  107

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Cys
        35                  40

<210>  108
<211>  44
<212>  PRT
<213>  Artificial Sequence

<220>
<223>

<400>  108

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Asp
        35                  40

<210>  109
<211>  44

<212>  PRT
<213>  Artificial Sequence

<220>
<223>

<400>  109

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Glu
        35                  40

<210>  110
<211>  44
<212>  PRT
<213>  Artificial Sequence

<220>
<223>

<400>  110

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Phe
        35                  40

<210>  111
<211>  44
<212>  PRT
<213>  Artificial Sequence

<220>
<223>

<400>  111

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Gly
        35                  40

<210>  112
<211>  44
<212>  PRT
<213>  Artificial Sequence

<220>
<223>

<400>  112

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly His
        35                  40

<210>  113
<211>  44
<212>  PRT
<213>  Artificial Sequence

<220>
<223>

<400>  113

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Ile
        35                  40

<210>  114
<211>  44
<212>  PRT
<213>  Artificial Sequence

<220>
<223>

<400>  114

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Lys
        35                  40

<210>  115
<211>  44
<212>  PRT
<213>  Artificial Sequence

<220>
<223>

<400>  115

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Leu
        35                  40

<210>  116
<211>  44
<212>  PRT
<213>  Artificial Sequence

<220>
<223>

<400>  116

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Met
        35                  40

<210>  117
<211>  44
<212>  PRT
<213>  Artificial Sequence

<220>
<223>

<400>  117

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro

                20                  25                  30

Asp Ala Ser Val Asn Phe Ser Glu Phe Ser Gly Asn
        35                  40

<210>  118
<211>  44
<212>  PRT
<213>  Artificial Sequence

<220>
<223>

<400>  118

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Pro
        35                  40

<210>  119
<211>  44
<212>  PRT
<213>  Artificial Sequence

<220>
<223>

<400>  119

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Gln
        35                  40

<210>  120
<211>  44
<212>  PRT
<213>  Artificial Sequence

<220>
<223>

<400>  120

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Arg
        35                  40

<210>  121
<211>  44
<212>  PRT
<213>  Artificial Sequence

<220>
<223>

<400>  121

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Ser
        35                  40

<210>  122
<211>  44
<212>  PRT
<213>  Artificial Sequence

<220>
<223>

<400>  122

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Thr
        35                  40

<210>  123
<211>  44
<212>  PRT
<213>  Artificial Sequence

<220>
<223>

<400>  123

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Val
        35                  40

<210>  124
<211>  44
<212>  PRT
<213>  Artificial Sequence

<220>
<223>

<400>  124

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Trp
        35                  40

<210>  125
<211>  44
<212>  PRT
<213>  Artificial Sequence

<220>
<223>

<400>  125

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gly Tyr
        35                  40

<210>  126
<211>  44
<212>  PRT
<213>  Artificial Sequence

<220>

<223>

<400>  126

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Ala
        35                  40

<210>  127
<211>  44
<212>  PRT
<213>  Artificial Sequence

<220>
<223>

<400>  127

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Cys
        35                  40

<210>  128
<211>  44
<212>  PRT
<213>  Artificial Sequence

<220>
<223>

<400>  128

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Asp
        35                  40

<210>  129
<211>  44
<212>  PRT

<213>  Artificial Sequence

<220>
<223>

<400>  129

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Glu
        35                  40

<210>  130
<211>  44
<212>  PRT
<213>  Artificial Sequence

<220>
<223>

<400>  130

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Phe
        35                  40

<210>  131
<211>  44
<212>  PRT
<213>  Artificial Sequence

<220>
<223>

<400>  131

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Gly
        35                  40

<210>  132
<211>  44
<212>  PRT
<213>  Artificial Sequence

<220>
<223>

<400>  132

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His His
        35                  40

<210>  133
<211>  44
<212>  PRT
<213>  Artificial Sequence

<220>
<223>

<400>  133

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Ile
        35                  40

<210>  134
<211>  44
<212>  PRT
<213>  Artificial Sequence

<220>
<223>

<400>  134

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Lys

35                  40

<210>  135
<211>  44
<212>  PRT
<213>  Artificial Sequence

<220>
<223>

<400>  135

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Leu
        35                  40

<210>  136
<211>  44
<212>  PRT
<213>  Artificial Sequence

<220>
<223>

<400>  136

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Met
        35                  40

<210>  137
<211>  44
<212>  PRT
<213>  Artificial Sequence

<220>
<223>

<400>  137

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Asn
        35                  40

<210>  138
<211>  44
<212>  PRT
<213>  Artificial Sequence

<220>
<223>

<400>  138

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Pro
        35                  40

<210>  139
<211>  44
<212>  PRT
<213>  Artificial Sequence

<220>
<223>

<400>  139

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Gln
        35                  40

<210>  140
<211>  44
<212>  PRT
<213>  Artificial Sequence

<220>
<223>

<400>  140

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Arg
        35                  40

<210>  141
<211>  44
<212>  PRT
<213>  Artificial Sequence

<220>
<223>

<400>  141

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Ser
        35                  40

<210>  142
<211>  44
<212>  PRT
<213>  Artificial Sequence

<220>
<223>

<400>  142

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Thr
        35                  40

<210>  143
<211>  44
<212>  PRT
<213>  Artificial Sequence

<220>
<223>

<400>  143

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Val
        35                  40

<210>  144
<211>  44
<212>  PRT
<213>  Artificial Sequence

<220>
<223>

<400>  144

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Trp
        35                  40

<210>  145
<211>  44
<212>  PRT
<213>  Artificial Sequence

<220>
<223>

<400>  145

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser His Tyr
        35                  40

<210>  146
<211>  44
<212>  PRT
<213>  Artificial Sequence

<220>
<223>

<400>  146

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Ala
        35                  40

<210>  147
<211>  44
<212>  PRT
<213>  Artificial Sequence

<220>
<223>

<400>  147

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Cys
        35                  40

<210>  148
<211>  44
<212>  PRT
<213>  Artificial Sequence

<220>
<223>

<400>  148

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Asp
        35                  40

<210>  149
<211>  44
<212>  PRT
<213>  Artificial Sequence

<220>
<223>

<400>  149

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Glu
        35                  40

<210>  150
<211>  44
<212>  PRT
<213>  Artificial Sequence

<220>
<223>

<400>  150

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Phe
        35                  40

<210>  151
<211>  44
<212>  PRT
<213>  Artificial Sequence

<220>
<223>

<400>  151

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Gly
        35                  40

<210>  152

<211>  44
<212>  PRT
<213>  Artificial Sequence

<220>
<223>

<400>  152

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile His
        35                  40

<210>  153
<211>  44
<212>  PRT
<213>  Artificial Sequence

<220>
<223>

<400>  153

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Ile
        35                  40

<210>  154
<211>  44
<212>  PRT
<213>  Artificial Sequence

<220>
<223>

<400>  154

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Lys
        35                  40

<210>  155
<211>  44
<212>  PRT
<213>  Artificial Sequence

<220>
<223>

<400>  155

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Leu
        35                  40

<210>  156
<211>  44
<212>  PRT
<213>  Artificial Sequence

<220>
<223>

<400>  156

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Met
        35                  40

<210>  157
<211>  44
<212>  PRT
<213>  Artificial Sequence

<220>
<223>

<400>  157

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Asn
        35                  40

<210>  158
<211>  44
<212>  PRT
<213>  Artificial Sequence

<220>
<223>

<400>  158

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Pro
        35                  40

<210>  159
<211>  44
<212>  PRT
<213>  Artificial Sequence

<220>
<223>

<400>  159

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Gln
        35                  40

<210>  160
<211>  44
<212>  PRT
<213>  Artificial Sequence

<220>
<223>

<400>  160

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Arg
        35                  40

<210>  161
<211>  44
<212>  PRT
<213>  Artificial Sequence

<220>
<223>

<400>  161

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Ser
        35                  40

<210>  162
<211>  44
<212>  PRT
<213>  Artificial Sequence

<220>
<223>

<400>  162

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Thr
        35                  40

<210>  163
<211>  44
<212>  PRT
<213>  Artificial Sequence

<220>
<223>

<400>  163

Met Gly Lys Gly Asp Pro Lys Lys Pro Arg Gly Lys Met Ser Ser Tyr

1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Val
        35                  40

<210>  164
<211>  44
<212>  PRT
<213>  Artificial Sequence

<220>
<223>

<400>  164

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Trp
        35                  40

<210>  165
<211>  44
<212>  PRT
<213>  Artificial Sequence

<220>
<223>

<400>  165

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ile Tyr
        35                  40

<210>  166
<211>  44
<212>  PRT
<213>  Artificial Sequence

<220>
<223>

<400>  166

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Cys
        35                  40

<210>  167
<211>  44
<212>  PRT
<213>  Artificial Sequence

<220>
<223>

<400>  167

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Asp
        35                  40

<210>  168
<211>  44
<212>  PRT
<213>  Artificial Sequence

<220>
<223>

<400>  168

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Phe
        35                  40

<210>  169
<211>  44
<212>  PRT
<213>  Artificial Sequence

<220>
<223>

<400>  169

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Gly
        35                  40

<210>  170
<211>  44
<212>  PRT
<213>  Artificial Sequence

<220>
<223>

<400>  170

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys His
        35                  40

<210>  171
<211>  44
<212>  PRT
<213>  Artificial Sequence

<220>
<223>

<400>  171

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Ile
        35                  40

<210>  172
<211>  44

<212>  PRT
<213>  Artificial Sequence

<220>
<223>

<400>  172

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Leu
        35                  40

<210>  173
<211>  44
<212>  PRT
<213>  Artificial Sequence

<220>
<223>

<400>  173

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Met
        35                  40

<210>  174
<211>  44
<212>  PRT
<213>  Artificial Sequence

<220>
<223>

<400>  174

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Asn
        35                  40

<210>  175
<211>  44
<212>  PRT
<213>  Artificial Sequence

<220>
<223>

<400>  175

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Gln
        35                  40

<210>  176
<211>  44
<212>  PRT
<213>  Artificial Sequence

<220>
<223>

<400>  176

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Arg
        35                  40

<210>  177
<211>  44
<212>  PRT
<213>  Artificial Sequence

<220>
<223>

<400>  177

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Ser
        35                  40

<210>  178
<211>  44
<212>  PRT
<213>  Artificial Sequence

<220>
<223>

<400>  178

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Thr
        35                  40

<210>  179
<211>  44
<212>  PRT
<213>  Artificial Sequence

<220>
<223>

<400>  179

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Val
        35                  40

<210>  180
<211>  44
<212>  PRT
<213>  Artificial Sequence

<220>
<223>

<400>  180

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro

            20                  25                  30

Asp Ala Ser Val Asn Phe Ser Glu Phe Ser Lys Trp
        35                  40

<210> 181
<211> 44
<212> PRT
<213> Artificial Sequence

<220>
<223>

<400> 181

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Tyr
        35                  40

<210> 182
<211> 44
<212> PRT
<213> Artificial Sequence

<220>
<223>

<400> 182

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Ala
        35                  40

<210> 183
<211> 44
<212> PRT
<213> Artificial Sequence

<220>
<223>

<400> 183

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Cys
        35                  40

<210>  184
<211>  44
<212>  PRT
<213>  Artificial Sequence

<220>
<223>

<400>  184

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Asp
        35                  40

<210>  185
<211>  44
<212>  PRT
<213>  Artificial Sequence

<220>
<223>

<400>  185

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Glu
        35                  40

<210>  186
<211>  44
<212>  PRT
<213>  Artificial Sequence

<220>
<223>

<400>  186

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Phe
        35                  40

<210>  187
<211>  44
<212>  PRT
<213>  Artificial Sequence

<220>
<223>

<400>  187

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Gly
        35                  40

<210>  188
<211>  44
<212>  PRT
<213>  Artificial Sequence

<220>
<223>

<400>  188

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu His
        35                  40

<210>  189
<211>  44
<212>  PRT
<213>  Artificial Sequence

<220>

<223>

<400>  189

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Ile
        35                  40

<210>  190
<211>  44
<212>  PRT
<213>  Artificial Sequence

<220>
<223>

<400>  190

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Lys
        35                  40

<210>  191
<211>  44
<212>  PRT
<213>  Artificial Sequence

<220>
<223>

<400>  191

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Leu
        35                  40

<210>  192
<211>  44
<212>  PRT

<213>  Artificial Sequence

<220>
<223>

<400>  192

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Met
        35                  40

<210>  193
<211>  44
<212>  PRT
<213>  Artificial Sequence

<220>
<223>

<400>  193

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Asn
        35                  40

<210>  194
<211>  44
<212>  PRT
<213>  Artificial Sequence

<220>
<223>

<400>  194

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Pro
        35                  40

<210>  195
<211>  44
<212>  PRT
<213>  Artificial Sequence

<220>
<223>

<400>  195

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Gln
        35                  40

<210>  196
<211>  44
<212>  PRT
<213>  Artificial Sequence

<220>
<223>

<400>  196

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Arg
        35                  40

<210>  197
<211>  44
<212>  PRT
<213>  Artificial Sequence

<220>
<223>

<400>  197

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Ser

35                  40

<210>  198
<211>  44
<212>  PRT
<213>  Artificial Sequence

<220>
<223>

<400>  198

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Thr
        35                  40

<210>  199
<211>  44
<212>  PRT
<213>  Artificial Sequence

<220>
<223>

<400>  199

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Val
        35                  40

<210>  200
<211>  44
<212>  PRT
<213>  Artificial Sequence

<220>
<223>

<400>  200

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Trp
        35                  40

<210>  201
<211>  44
<212>  PRT
<213>  Artificial Sequence

<220>
<223>

<400>  201

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Leu Tyr
        35                  40

<210>  202
<211>  44
<212>  PRT
<213>  Artificial Sequence

<220>
<223>

<400>  202

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Ala
        35                  40

<210>  203
<211>  44
<212>  PRT
<213>  Artificial Sequence

<220>
<223>

<400>  203

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Cys
        35                  40

<210>  204
<211>  44
<212>  PRT
<213>  Artificial Sequence

<220>
<223>

<400>  204

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Asp
        35                  40

<210>  205
<211>  44
<212>  PRT
<213>  Artificial Sequence

<220>
<223>

<400>  205

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Glu
        35                  40

<210>  206
<211>  44
<212>  PRT
<213>  Artificial Sequence

<220>
<223>

<400>  206

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Phe
        35                  40

<210>  207
<211>  44
<212>  PRT
<213>  Artificial Sequence

<220>
<223>

<400>  207

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Gly
        35                  40

<210>  208
<211>  44
<212>  PRT
<213>  Artificial Sequence

<220>
<223>

<400>  208

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met His
        35                  40

<210>  209
<211>  44
<212>  PRT
<213>  Artificial Sequence

<220>
<223>

<400>  209

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Ile
        35                  40

<210>  210
<211>  44
<212>  PRT
<213>  Artificial Sequence

<220>
<223>

<400>  210

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Lys
        35                  40

<210>  211
<211>  44
<212>  PRT
<213>  Artificial Sequence

<220>
<223>

<400>  211

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Leu
        35                  40

<210>  212
<211>  44
<212>  PRT
<213>  Artificial Sequence

<220>
<223>

<400>  212

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Met
        35                  40

<210>  213
<211>  44
<212>  PRT
<213>  Artificial Sequence

<220>
<223>

<400>  213

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Asn
        35                  40

<210>  214
<211>  44
<212>  PRT
<213>  Artificial Sequence

<220>
<223>

<400>  214

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Pro
        35                  40

<210>  215

<211>  44
<212>  PRT
<213>  Artificial Sequence

<220>
<223>

<400>  215

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Gln
        35                  40

<210>  216
<211>  44
<212>  PRT
<213>  Artificial Sequence

<220>
<223>

<400>  216

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Arg
        35                  40

<210>  217
<211>  44
<212>  PRT
<213>  Artificial Sequence

<220>
<223>

<400>  217

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Ser
        35                  40

<210>  218
<211>  44
<212>  PRT
<213>  Artificial Sequence

<220>
<223>

<400>  218

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Thr
        35                  40

<210>  219
<211>  44
<212>  PRT
<213>  Artificial Sequence

<220>
<223>

<400>  219

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Val
        35                  40

<210>  220
<211>  44
<212>  PRT
<213>  Artificial Sequence

<220>
<223>

<400>  220

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Trp
        35                  40

<210>  221
<211>  44
<212>  PRT
<213>  Artificial Sequence

<220>
<223>

<400>  221

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Met Tyr
        35                  40

<210>  222
<211>  44
<212>  PRT
<213>  Artificial Sequence

<220>
<223>

<400>  222

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Ala
        35                  40

<210>  223
<211>  44
<212>  PRT
<213>  Artificial Sequence

<220>
<223>

<400>  223

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Cys
        35                  40

<210>  224
<211>  44
<212>  PRT
<213>  Artificial Sequence

<220>
<223>

<400>  224

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Asp
        35                  40

<210>  225
<211>  44
<212>  PRT
<213>  Artificial Sequence

<220>
<223>

<400>  225

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Glu
        35                  40

<210>  226
<211>  44
<212>  PRT
<213>  Artificial Sequence

<220>
<223>

<400>  226

Met Gly Lys Gly Asp Pro Lys Lys Pro Arg Gly Lys Met Ser Ser Tyr

1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Phe
        35                  40

<210>  227
<211>  44
<212>  PRT
<213>  Artificial Sequence

<220>
<223>

<400>  227

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Gly
        35                  40

<210>  228
<211>  44
<212>  PRT
<213>  Artificial Sequence

<220>
<223>

<400>  228

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn His
        35                  40

<210>  229
<211>  44
<212>  PRT
<213>  Artificial Sequence

<220>
<223>

<400>  229

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Ile
        35                  40

<210>  230
<211>  44
<212>  PRT
<213>  Artificial Sequence

<220>
<223>

<400>  230

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Lys
        35                  40

<210>  231
<211>  44
<212>  PRT
<213>  Artificial Sequence

<220>
<223>

<400>  231

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Leu
        35                  40

<210>  232
<211>  44
<212>  PRT
<213>  Artificial Sequence

<220>
<223>

<400>  232

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Met
        35                  40

<210>  233
<211>  44
<212>  PRT
<213>  Artificial Sequence

<220>
<223>

<400>  233

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Asn
        35                  40

<210>  234
<211>  44
<212>  PRT
<213>  Artificial Sequence

<220>
<223>

<400>  234

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Pro
        35                  40

<210>  235
<211>  44

<212>  PRT
<213>  Artificial Sequence

<220>
<223>

<400>  235

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Gln
        35                  40

<210>  236
<211>  44
<212>  PRT
<213>  Artificial Sequence

<220>
<223>

<400>  236

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Arg
        35                  40

<210>  237
<211>  44
<212>  PRT
<213>  Artificial Sequence

<220>
<223>

<400>  237

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Ser
        35                  40

<210>  238
<211>  44
<212>  PRT
<213>  Artificial Sequence

<220>
<223>

<400>  238

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Thr
        35                  40

<210>  239
<211>  44
<212>  PRT
<213>  Artificial Sequence

<220>
<223>

<400>  239

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Val
        35                  40

<210>  240
<211>  44
<212>  PRT
<213>  Artificial Sequence

<220>
<223>

<400>  240

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Trp
        35                  40

<210>  241
<211>  44
<212>  PRT
<213>  Artificial Sequence

<220>
<223>

<400>  241

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Asn Tyr
        35                  40

<210>  242
<211>  44
<212>  PRT
<213>  Artificial Sequence

<220>
<223>

<400>  242

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Ala
        35                  40

<210>  243
<211>  44
<212>  PRT
<213>  Artificial Sequence

<220>
<223>

<400>  243

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro

20                  25                  30

Asp Ala Ser Val Asn Phe Ser Glu Phe Ser Pro Cys
        35                  40

<210>  244
<211>  44
<212>  PRT
<213>  Artificial Sequence

<220>
<223>

<400>  244

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Asp
        35                  40

<210>  245
<211>  44
<212>  PRT
<213>  Artificial Sequence

<220>
<223>

<400>  245

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Glu
        35                  40

<210>  246
<211>  44
<212>  PRT
<213>  Artificial Sequence

<220>
<223>

<400>  246

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Phe
        35                  40

<210>  247
<211>  44
<212>  PRT
<213>  Artificial Sequence

<220>
<223>

<400>  247

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Gly
        35                  40

<210>  248
<211>  44
<212>  PRT
<213>  Artificial Sequence

<220>
<223>

<400>  248

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro His
        35                  40

<210>  249
<211>  44
<212>  PRT
<213>  Artificial Sequence

<220>
<223>

<400>  249

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Ile
        35                  40

<210>  250
<211>  44
<212>  PRT
<213>  Artificial Sequence

<220>
<223>

<400>  250

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Leu
        35                  40

<210>  251
<211>  44
<212>  PRT
<213>  Artificial Sequence

<220>
<223>

<400>  251

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Met
        35                  40

<210>  252
<211>  44
<212>  PRT
<213>  Artificial Sequence

<220>

<223>

<400>  252

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Asn
        35                  40

<210>  253
<211>  44
<212>  PRT
<213>  Artificial Sequence

<220>
<223>

<400>  253

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Gln
        35                  40

<210>  254
<211>  44
<212>  PRT
<213>  Artificial Sequence

<220>
<223>

<400>  254

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Arg
        35                  40

<210>  255
<211>  44
<212>  PRT

<213>  Artificial Sequence

<220>
<223>

<400>  255

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Ser
        35                  40

<210>  256
<211>  44
<212>  PRT
<213>  Artificial Sequence

<220>
<223>

<400>  256

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Thr
        35                  40

<210>  257
<211>  44
<212>  PRT
<213>  Artificial Sequence

<220>
<223>

<400>  257

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Val
        35                  40

<210>  258
<211>  44
<212>  PRT
<213>  Artificial Sequence

<220>
<223>

<400>  258

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Trp
        35                  40

<210>  259
<211>  44
<212>  PRT
<213>  Artificial Sequence

<220>
<223>

<400>  259

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Pro Tyr
        35                  40

<210>  260
<211>  44
<212>  PRT
<213>  Artificial Sequence

<220>
<223>

<400>  260

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Ala

35                  40


<210>  261
<211>  44
<212>  PRT
<213>  Artificial Sequence

<220>
<223>

<400>  261

Met Gly Lys Gly Asp Pro Lys Lys Pro Arg Gly Lys Met Ser Ser Tyr
1               5                   10                  15


Ala Phe Phe Val Gln Thr Cys Arg Glu Glu His Lys Lys Lys His Pro
            20                  25                  30


Asp Ala Ser Val Asn Phe Ser Glu Phe Ser Gln Cys
        35                  40


<210>  262
<211>  44
<212>  PRT
<213>  Artificial Sequence

<220>
<223>

<400>  262

Met Gly Lys Gly Asp Pro Lys Lys Pro Arg Gly Lys Met Ser Ser Tyr
1               5                   10                  15


Ala Phe Phe Val Gln Thr Cys Arg Glu Glu His Lys Lys Lys His Pro
            20                  25                  30


Asp Ala Ser Val Asn Phe Ser Glu Phe Ser Gln Asp
        35                  40


<210>  263
<211>  44
<212>  PRT
<213>  Artificial Sequence

<220>
<223>

<400>  263

Met Gly Lys Gly Asp Pro Lys Lys Pro Arg Gly Lys Met Ser Ser Tyr
1               5                   10                  15


Ala Phe Phe Val Gln Thr Cys Arg Glu Glu His Lys Lys Lys His Pro
            20                  25                  30

Asp Ala Ser Val Asn Phe Ser Glu Phe Ser Gln Glu
        35                  40

<210>  264
<211>  44
<212>  PRT
<213>  Artificial Sequence

<220>
<223>

<400>  264

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Phe
        35                  40

<210>  265
<211>  44
<212>  PRT
<213>  Artificial Sequence

<220>
<223>

<400>  265

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Gly
        35                  40

<210>  266
<211>  44
<212>  PRT
<213>  Artificial Sequence

<220>
<223>

<400>  266

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln His
        35                  40

<210>  267
<211>  44
<212>  PRT
<213>  Artificial Sequence

<220>
<223>

<400>  267

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Ile
        35                  40

<210>  268
<211>  44
<212>  PRT
<213>  Artificial Sequence

<220>
<223>

<400>  268

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Lys
        35                  40

<210>  269
<211>  44
<212>  PRT
<213>  Artificial Sequence

<220>
<223>

<400>  269

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Leu
        35                  40

<210>  270
<211>  44
<212>  PRT
<213>  Artificial Sequence

<220>
<223>

<400>  270

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Met
        35                  40

<210>  271
<211>  44
<212>  PRT
<213>  Artificial Sequence

<220>
<223>

<400>  271

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Asn
        35                  40

<210>  272
<211>  44
<212>  PRT
<213>  Artificial Sequence

<220>
<223>

<400>  272

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Pro
        35                  40

<210>  273
<211>  44
<212>  PRT
<213>  Artificial Sequence

<220>
<223>

<400>  273

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Gln
        35                  40

<210>  274
<211>  44
<212>  PRT
<213>  Artificial Sequence

<220>
<223>

<400>  274

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Arg
        35                  40

<210>  275
<211>  44
<212>  PRT
<213>  Artificial Sequence

<220>
<223>

<400>  275

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Ser
        35                  40

<210>  276
<211>  44
<212>  PRT
<213>  Artificial Sequence

<220>
<223>

<400>  276

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Thr
        35                  40

<210>  277
<211>  44
<212>  PRT
<213>  Artificial Sequence

<220>
<223>

<400>  277

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Val
        35                  40

<210>  278

<211>  44
<212>  PRT
<213>  Artificial Sequence

<220>
<223>

<400>  278

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Trp
        35                  40

<210>  279
<211>  44
<212>  PRT
<213>  Artificial Sequence

<220>
<223>

<400>  279

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Gln Tyr
        35                  40

<210>  280
<211>  44
<212>  PRT
<213>  Artificial Sequence

<220>
<223>

<400>  280

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Ala
        35                  40

<210>  281
<211>  44
<212>  PRT
<213>  Artificial Sequence

<220>
<223>

<400>  281

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Cys
        35                  40

<210>  282
<211>  44
<212>  PRT
<213>  Artificial Sequence

<220>
<223>

<400>  282

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Asp
        35                  40

<210>  283
<211>  44
<212>  PRT
<213>  Artificial Sequence

<220>
<223>

<400>  283

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Glu
        35                  40

<210>  284
<211>  44
<212>  PRT
<213>  Artificial Sequence

<220>
<223>

<400>  284

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Phe
        35                  40

<210>  285
<211>  44
<212>  PRT
<213>  Artificial Sequence

<220>
<223>

<400>  285

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Gly
        35                  40

<210>  286
<211>  44
<212>  PRT
<213>  Artificial Sequence

<220>
<223>

<400>  286

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg His
        35                  40

<210>  287
<211>  44
<212>  PRT
<213>  Artificial Sequence

<220>
<223>

<400>  287

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Ile
        35                  40

<210>  288
<211>  44
<212>  PRT
<213>  Artificial Sequence

<220>
<223>

<400>  288

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Lys
        35                  40

<210>  289
<211>  44
<212>  PRT
<213>  Artificial Sequence

<220>
<223>

<400>  289

Met Gly Lys Gly Asp Pro Lys Lys Pro Arg Gly Lys Met Ser Ser Tyr

1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Leu
        35                  40

<210>  290
<211>  44
<212>  PRT
<213>  Artificial Sequence

<220>
<223>

<400>  290

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Met
        35                  40

<210>  291
<211>  44
<212>  PRT
<213>  Artificial Sequence

<220>
<223>

<400>  291

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Asn
        35                  40

<210>  292
<211>  44
<212>  PRT
<213>  Artificial Sequence

<220>
<223>

<400>  292

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Pro
        35                  40

<210>  293
<211>  44
<212>  PRT
<213>  Artificial Sequence

<220>
<223>

<400>  293

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Gln
        35                  40

<210>  294
<211>  44
<212>  PRT
<213>  Artificial Sequence

<220>
<223>

<400>  294

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Arg
        35                  40

<210>  295
<211>  44
<212>  PRT
<213>  Artificial Sequence

<220>
<223>

<400>  295

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Ser
        35                  40

<210>  296
<211>  44
<212>  PRT
<213>  Artificial Sequence

<220>
<223>

<400>  296

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Thr
        35                  40

<210>  297
<211>  44
<212>  PRT
<213>  Artificial Sequence

<220>
<223>

<400>  297

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Val
        35                  40

<210>  298
<211>  44

<212>  PRT
<213>  Artificial Sequence

<220>
<223>

<400>  298

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Trp
        35                  40

<210>  299
<211>  44
<212>  PRT
<213>  Artificial Sequence

<220>
<223>

<400>  299

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Arg Tyr
        35                  40

<210>  300
<211>  44
<212>  PRT
<213>  Artificial Sequence

<220>
<223>

<400>  300

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Ala
        35                  40

<210>  301
<211>  44
<212>  PRT
<213>  Artificial Sequence

<220>
<223>

<400>  301

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Cys
        35                  40

<210>  302
<211>  44
<212>  PRT
<213>  Artificial Sequence

<220>
<223>

<400>  302

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Asp
        35                  40

<210>  303
<211>  44
<212>  PRT
<213>  Artificial Sequence

<220>
<223>

<400>  303

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Glu
        35                  40

<210>  304
<211>  44
<212>  PRT
<213>  Artificial Sequence

<220>
<223>

<400>  304

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Phe
        35                  40

<210>  305
<211>  44
<212>  PRT
<213>  Artificial Sequence

<220>
<223>

<400>  305

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Gly
        35                  40

<210>  306
<211>  44
<212>  PRT
<213>  Artificial Sequence

<220>
<223>

<400>  306

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro

20                  25                  30

Asp Ala Ser Val Asn Phe Ser Glu Phe Ser Ser His
        35                  40

<210>  307
<211>  44
<212>  PRT
<213>  Artificial Sequence

<220>
<223>

<400>  307

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Ile
        35                  40

<210>  308
<211>  44
<212>  PRT
<213>  Artificial Sequence

<220>
<223>

<400>  308

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Lys
        35                  40

<210>  309
<211>  44
<212>  PRT
<213>  Artificial Sequence

<220>
<223>

<400>  309

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Leu
        35                  40

<210>  310
<211>  44
<212>  PRT
<213>  Artificial Sequence

<220>
<223>

<400>  310

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Met
        35                  40

<210>  311
<211>  44
<212>  PRT
<213>  Artificial Sequence

<220>
<223>

<400>  311

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Asn
        35                  40

<210>  312
<211>  44
<212>  PRT
<213>  Artificial Sequence

<220>
<223>

<400>  312

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Pro
        35                  40

<210>  313
<211>  44
<212>  PRT
<213>  Artificial Sequence

<220>
<223>

<400>  313

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Gln
        35                  40

<210>  314
<211>  44
<212>  PRT
<213>  Artificial Sequence

<220>
<223>

<400>  314

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Arg
        35                  40

<210>  315
<211>  44
<212>  PRT
<213>  Artificial Sequence

<220>

<223>

<400>  315

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Ser
        35                  40

<210>  316
<211>  44
<212>  PRT
<213>  Artificial Sequence

<220>
<223>

<400>  316

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Thr
        35                  40

<210>  317
<211>  44
<212>  PRT
<213>  Artificial Sequence

<220>
<223>

<400>  317

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Val
        35                  40

<210>  318
<211>  44
<212>  PRT

<213>  Artificial Sequence

<220>
<223>

<400>  318

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Trp
        35                  40

<210>  319
<211>  44
<212>  PRT
<213>  Artificial Sequence

<220>
<223>

<400>  319

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Ser Tyr
        35                  40

<210>  320
<211>  44
<212>  PRT
<213>  Artificial Sequence

<220>
<223>

<400>  320

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Ala
        35                  40

<210>  321
<211>  44
<212>  PRT
<213>  Artificial Sequence

<220>
<223>

<400>  321

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Cys
        35                  40

<210>  322
<211>  44
<212>  PRT
<213>  Artificial Sequence

<220>
<223>

<400>  322

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Asp
        35                  40

<210>  323
<211>  44
<212>  PRT
<213>  Artificial Sequence

<220>
<223>

<400>  323

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Glu

35                  40

<210>  324
<211>  44
<212>  PRT
<213>  Artificial Sequence

<220>
<223>

<400>  324

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Phe
        35                  40

<210>  325
<211>  44
<212>  PRT
<213>  Artificial Sequence

<220>
<223>

<400>  325

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Gly
        35                  40

<210>  326
<211>  44
<212>  PRT
<213>  Artificial Sequence

<220>
<223>

<400>  326

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr His
        35                  40

<210>  327
<211>  44
<212>  PRT
<213>  Artificial Sequence

<220>
<223>

<400>  327

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Ile
        35                  40

<210>  328
<211>  44
<212>  PRT
<213>  Artificial Sequence

<220>
<223>

<400>  328

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Lys
        35                  40

<210>  329
<211>  44
<212>  PRT
<213>  Artificial Sequence

<220>
<223>

<400>  329

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Leu
        35                  40

<210>  330
<211>  44
<212>  PRT
<213>  Artificial Sequence

<220>
<223>

<400>  330

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Met
        35                  40

<210>  331
<211>  44
<212>  PRT
<213>  Artificial Sequence

<220>
<223>

<400>  331

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Asn
        35                  40

<210>  332
<211>  44
<212>  PRT
<213>  Artificial Sequence

<220>
<223>

<400>  332

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Pro
        35                  40

<210>  333
<211>  44
<212>  PRT
<213>  Artificial Sequence

<220>
<223>

<400>  333

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Gln
        35                  40

<210>  334
<211>  44
<212>  PRT
<213>  Artificial Sequence

<220>
<223>

<400>  334

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Arg
        35                  40

<210>  335
<211>  44
<212>  PRT
<213>  Artificial Sequence

<220>
<223>

<400>  335

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Ser
        35                  40

<210>  336
<211>  44
<212>  PRT
<213>  Artificial Sequence

<220>
<223>

<400>  336

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Thr
        35                  40

<210>  337
<211>  44
<212>  PRT
<213>  Artificial Sequence

<220>
<223>

<400>  337

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Val
        35                  40

<210>  338
<211>  44
<212>  PRT
<213>  Artificial Sequence

<220>
<223>

<400>  338

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Trp
        35                  40

<210>  339
<211>  44
<212>  PRT
<213>  Artificial Sequence

<220>
<223>

<400>  339

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Thr Tyr
        35                  40

<210>  340
<211>  44
<212>  PRT
<213>  Artificial Sequence

<220>
<223>

<400>  340

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Ala
        35                  40

<210>  341

<211>  44
<212>  PRT
<213>  Artificial Sequence

<220>
<223>

<400>  341

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Cys
        35                  40

<210>  342
<211>  44
<212>  PRT
<213>  Artificial Sequence

<220>
<223>

<400>  342

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Asp
        35                  40

<210>  343
<211>  44
<212>  PRT
<213>  Artificial Sequence

<220>
<223>

<400>  343

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Glu
        35                  40

<210>  344
<211>  44
<212>  PRT
<213>  Artificial Sequence

<220>
<223>

<400>  344

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Phe
        35                  40

<210>  345
<211>  44
<212>  PRT
<213>  Artificial Sequence

<220>
<223>

<400>  345

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Gly
        35                  40

<210>  346
<211>  44
<212>  PRT
<213>  Artificial Sequence

<220>
<223>

<400>  346

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val His
        35                  40

<210>  347
<211>  44
<212>  PRT
<213>  Artificial Sequence

<220>
<223>

<400>  347

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Ile
        35                  40

<210>  348
<211>  44
<212>  PRT
<213>  Artificial Sequence

<220>
<223>

<400>  348

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Lys
        35                  40

<210>  349
<211>  44
<212>  PRT
<213>  Artificial Sequence

<220>
<223>

<400>  349

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Leu
        35                  40

<210>  350
<211>  44
<212>  PRT
<213>  Artificial Sequence

<220>
<223>

<400>  350

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Met
        35                  40

<210>  351
<211>  44
<212>  PRT
<213>  Artificial Sequence

<220>
<223>

<400>  351

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Asn
        35                  40

<210>  352
<211>  44
<212>  PRT
<213>  Artificial Sequence

<220>
<223>

<400>  352

Met Gly Lys Gly Asp Pro Lys Lys Pro Arg Gly Lys Met Ser Ser Tyr

1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Pro
        35                  40

<210>  353
<211>  44
<212>  PRT
<213>  Artificial Sequence

<220>
<223>

<400>  353

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Gln
        35                  40

<210>  354
<211>  44
<212>  PRT
<213>  Artificial Sequence

<220>
<223>

<400>  354

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Arg
        35                  40

<210>  355
<211>  44
<212>  PRT
<213>  Artificial Sequence

<220>
<223>

<400>  355

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Ser
        35                  40

<210>  356
<211>  44
<212>  PRT
<213>  Artificial Sequence

<220>
<223>

<400>  356

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Thr
        35                  40

<210>  357
<211>  44
<212>  PRT
<213>  Artificial Sequence

<220>
<223>

<400>  357

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Val
        35                  40

<210>  358
<211>  44
<212>  PRT
<213>  Artificial Sequence

<220>
<223>

<400>  358

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Trp
        35                  40

<210>  359
<211>  44
<212>  PRT
<213>  Artificial Sequence

<220>
<223>

<400>  359

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Val Tyr
        35                  40

<210>  360
<211>  44
<212>  PRT
<213>  Artificial Sequence

<220>
<223>

<400>  360

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Ala
        35                  40

<210>  361
<211>  44

<212>  PRT
<213>  Artificial Sequence

<220>
<223>

<400>  361

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Cys
        35                  40

<210>  362
<211>  44
<212>  PRT
<213>  Artificial Sequence

<220>
<223>

<400>  362

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Asp
        35                  40

<210>  363
<211>  44
<212>  PRT
<213>  Artificial Sequence

<220>
<223>

<400>  363

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Glu
        35                  40

<210>  364
<211>  44
<212>  PRT
<213>  Artificial Sequence

<220>
<223>

<400>  364

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Phe
        35                  40

<210>  365
<211>  44
<212>  PRT
<213>  Artificial Sequence

<220>
<223>

<400>  365

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Gly
        35                  40

<210>  366
<211>  44
<212>  PRT
<213>  Artificial Sequence

<220>
<223>

<400>  366

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp His
        35                  40

<210>  367
<211>  44
<212>  PRT
<213>  Artificial Sequence

<220>
<223>

<400>  367

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Ile
        35                  40

<210>  368
<211>  44
<212>  PRT
<213>  Artificial Sequence

<220>
<223>

<400>  368

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Lys
        35                  40

<210>  369
<211>  44
<212>  PRT
<213>  Artificial Sequence

<220>
<223>

<400>  369

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro

20                  25                  30

Asp Ala Ser Val Asn Phe Ser Glu Phe Ser Trp Leu
        35                  40

<210>  370
<211>  44
<212>  PRT
<213>  Artificial Sequence

<220>
<223>

<400>  370

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Met
        35                  40

<210>  371
<211>  44
<212>  PRT
<213>  Artificial Sequence

<220>
<223>

<400>  371

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Asn
        35                  40

<210>  372
<211>  44
<212>  PRT
<213>  Artificial Sequence

<220>
<223>

<400>  372

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Pro
        35                  40

<210>  373
<211>  44
<212>  PRT
<213>  Artificial Sequence

<220>
<223>

<400>  373

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Gln
        35                  40

<210>  374
<211>  44
<212>  PRT
<213>  Artificial Sequence

<220>
<223>

<400>  374

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Arg
        35                  40

<210>  375
<211>  44
<212>  PRT
<213>  Artificial Sequence

<220>
<223>

<400>  375

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Ser
        35                  40

<210>  376
<211>  44
<212>  PRT
<213>  Artificial Sequence

<220>
<223>

<400>  376

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Thr
        35                  40

<210>  377
<211>  44
<212>  PRT
<213>  Artificial Sequence

<220>
<223>

<400>  377

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Val
        35                  40

<210>  378
<211>  44
<212>  PRT
<213>  Artificial Sequence

<220>

<223>

<400>  378

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Trp
        35                  40

<210>  379
<211>  44
<212>  PRT
<213>  Artificial Sequence

<220>
<223>

<400>  379

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Trp Tyr
        35                  40

<210>  380
<211>  44
<212>  PRT
<213>  Artificial Sequence

<220>
<223>

<400>  380

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Ala
        35                  40

<210>  381
<211>  44
<212>  PRT

<213>  Artificial Sequence

<220>
<223>

<400>  381

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Cys
        35                  40

<210>  382
<211>  44
<212>  PRT
<213>  Artificial Sequence

<220>
<223>

<400>  382

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Asp
        35                  40

<210>  383
<211>  44
<212>  PRT
<213>  Artificial Sequence

<220>
<223>

<400>  383

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Glu
        35                  40

<210>  384
<211>  44
<212>  PRT
<213>  Artificial Sequence

<220>
<223>

<400>  384

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Phe
        35                  40

<210>  385
<211>  44
<212>  PRT
<213>  Artificial Sequence

<220>
<223>

<400>  385

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Gly
        35                  40

<210>  386
<211>  44
<212>  PRT
<213>  Artificial Sequence

<220>
<223>

<400>  386

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr His

35                  40

<210>  387
<211>  44
<212>  PRT
<213>  Artificial Sequence

<220>
<223>

<400>  387

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Ile
        35                  40

<210>  388
<211>  44
<212>  PRT
<213>  Artificial Sequence

<220>
<223>

<400>  388

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Lys
        35                  40

<210>  389
<211>  44
<212>  PRT
<213>  Artificial Sequence

<220>
<223>

<400>  389

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Leu
        35                  40

<210>  390
<211>  44
<212>  PRT
<213>  Artificial Sequence

<220>
<223>

<400>  390

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Met
        35                  40

<210>  391
<211>  44
<212>  PRT
<213>  Artificial Sequence

<220>
<223>

<400>  391

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Asn
        35                  40

<210>  392
<211>  44
<212>  PRT
<213>  Artificial Sequence

<220>
<223>

<400>  392

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Pro
        35                  40

<210>  393
<211>  44
<212>  PRT
<213>  Artificial Sequence

<220>
<223>

<400>  393

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Gln
        35                  40

<210>  394
<211>  44
<212>  PRT
<213>  Artificial Sequence

<220>
<223>

<400>  394

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Arg
        35                  40

<210>  395
<211>  44
<212>  PRT
<213>  Artificial Sequence

<220>
<223>

<400>  395

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Ser
        35                  40

<210>  396
<211>  44
<212>  PRT
<213>  Artificial Sequence

<220>
<223>

<400>  396

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Thr
        35                  40

<210>  397
<211>  44
<212>  PRT
<213>  Artificial Sequence

<220>
<223>

<400>  397

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Val
        35                  40

<210>  398
<211>  44
<212>  PRT
<213>  Artificial Sequence

<220>
<223>

<400>  398

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Trp
        35                  40

<210>  399
<211>  44
<212>  PRT
<213>  Artificial Sequence

<220>
<223>

<400>  399

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Tyr Tyr
        35                  40

<210>  400
<211>  43
<212>  PRT
<213>  Artificial Sequence

<220>
<223>

<400>  400

Gly Lys Gly Asp Pro Lys Lys Pro Arg Gly Lys Met Ser Ser Tyr Ala
1               5                   10                  15

Phe Phe Val Gln Thr Cys Arg Glu Glu His Lys Lys Lys His Pro Asp
            20                  25                  30

Ala Ser Val Asn Phe Ser Glu Phe Ser Lys Lys
        35                  40

<210>  401
<211>  215
<212>  PRT
<213>  Homo sapiens

<400>  401

<210>  402
<211>  44
<212>  PRT
<213>  Artificial Sequence

<220>
<223>

<400>  402

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Lys
        35                  40

<210>  403
<211>  43
<212>  PRT
<213>  Artificial Sequence

<220>
<223>

<400>  403

Gly Lys Gly Asp Pro Lys Lys Pro Arg Gly Lys Met Ser Ser Tyr Ala
1               5                   10                  15

Phe Phe Val Gln Thr Cys Arg Glu Glu His Lys Lys Lys His Pro Asp
            20                  25                  30

Ala Ser Val Asn Phe Ser Glu Phe Ser Pro Pro
        35                  40